# EUROPEAN PATENT SPECIFICATION

(11) **EP 3 831 276 B1**
(45) Date of publication and mention of the grant of the patent: **30.09.2026**
(21) Application number: 18928715.4
(22) Date of filing: 01.08.2018
(51) Int. Cl.: A61B 5/00, A61B 5/01, A61B 5/021, A61B 5/08, A61B 5/145

(54) **MONITOR AND CONTROL METHOD THEREFOR, COLLECTING AND RECEIVING DEVICES AND COMMUNICATION METHOD THEREFOR**
MONITOR UND STEUERVERFAHREN DAFÜR, SAMMEL- UND EMPFANGSVORRICHTUNGEN UND KOMMUNIKATIONSVERFAHREN DAFÜR
MONITEUR ET MÉTHODE DE CONTRÔLE ASSOCIÉE, DISPOSITIFS DE COLLECTE ET DE RÉCEPTION ET MÉTHODE DE COMMUNICATION ASSOCIÉE

(43) Date of publication of application: 09.06.2021
(73) Proprietor: Shenzhen Mindray Bio-Medical Electronics Co., Ltd., Shenzhen, Guangdong 518057 (CN); Shenzhen Mindray Scientific Co., Ltd., Shenzhen, Guangdong 518000 (CN)
(72) Inventor: LIU, Qiling, Shenzhen, Guangdong 518057 (CN); REN, Jian, Shenzhen, Guangdong 518057 (CN); FU, Neng, Shenzhen, Guangdong 518057 (CN); NIE, Pengpeng, Shenzhen, Guangdong 518057 (CN); LIU, Zhonghua, Shenzhen, Guangdong 518057 (CN)
(74) Representative: KIPA AB
(86) International application number: PCT/CN2018/098096
(87) International publication number: WO 2020/024168

(56) References cited:
- WO-A1-2018/022566
- CN-A- 101 869 469
- CN-A- 103 169 448
- CN-A- 106 658 362
- US-A1- 2004 260 363
- US-A1- 2009 222 659
- US-A1- 2013 029 596
- US-A1- 2017 172 415
- US-B1- 9 463 325
- HAATAJA K ET AL: "Two practical man-in-the-middle attacks on Bluetooth secure simple pairing and countermeasures", IEEE TRANSACTIONS ON WIRELESS COMMUNICATIONS, IEEE SERVICE CENTER, PISCATAWAY, NJ, US, vol. 9, no. 1, 1 January 2010 (2010-01-01), pages 384 - 392, XP011299484, ISSN: 1536-1276

## Description

### TECHNICAL FIELD

The disclosure relates to the field of monitors, and in particular to a monitor and a control method therefor, collecting and receiving devices and a communication method therefor.

### BACKGROUND

In a conventional bedside patient monitoring system, a variety of physiological parameters such as ECG, SpO2, TEMP, NIBP, RESP, and IBP may be measured for one patient. Every kind of physiological parameter needs to be measured by connecting a corresponding sensor and cables to a monitor or a multi-parameter module. For a patient with relatively more physiological parameters that need to be monitored, there will be a plurality of cables connecting the patient to the monitor. In this case, the patient is confined to a small range around the monitor, and the restraint of the plurality of cables will cause many inconveniences to the patient with mobility.

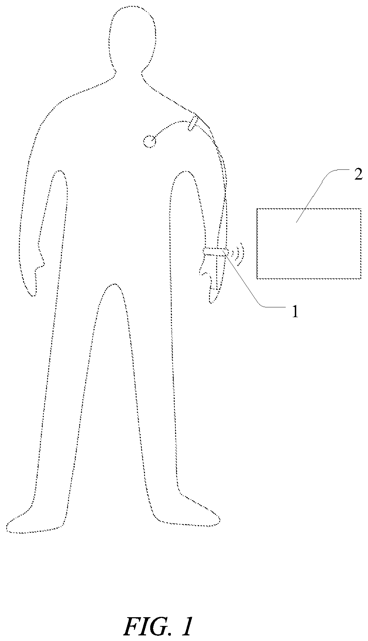

As shown in FIG. 1, although a telemetry product 1 for monitoring physiological parameters of a patient in a wireless manner has appeared at present, such products can only monitor a single type of physiological parameters, for example, can only measure one or two parameters of ECG, SpO2, NIBP, and RESP, which cannot satisfy the patient with a plurality of parameters that needs to be monitored. In addition, such a system generally can only send data to a remote central station 2 in a wireless manner for centralized display and processing, which is not convenient for medical personnel at the bedside to operate and observe waveforms. When the central station 2 is interfered by the surrounding environment or a wireless signal is poor, the central station cannot receive measurement data. If a physiological state of the patient changes, the medical personnel cannot find and intervene in time, which may delay the treatment.

For the current telemetry product 1, although the restraint of a conventional bedside monitoring apparatus on the patient can be alleviated, a connection between the telemetry product and the central station 2 is fixed. That is, if there are a telemetry system A and a telemetry system B in a hospital, apparatuses originally connected to the systems A and B respectively cannot be connected to each other conveniently for use. Moreover, even if a new telemetry apparatus needs to be added to a same telemetry system, the apparatus cannot be connected to the system conveniently, which requires complicated operation steps, and affects working efficiency of the medical personnel. In other words, when a plurality of telemetry products 1 of different systems or a plurality of telemetry products 1 of a same system are paired with the same central station 2, the process is complicated, the pairing relationship is easily interfered and damaged, and the data communication is easily interfered, thereby reducing the working efficiency of the medical personnel and integrity and stability of measurement data.

WO2018/022566 discloses a system facilitating telemetry data communication security between an implantable device and an external clinical device. The implantable device and the external clinical device generate security information based on a clinical telemetry session request. The implantable device communicates with the external clinical device via advertising to communicate the security information.

US2009/0222659 discloses a communication device for performing communication by employing first and second communication units. The devices uses the NFC communication to obtain the Bluetooth information, and transmit and receive the public keys and the data information using the Bluetooth communication.

Haataja K ET (titled "Two practical main-in-the-middle attacks on Bluetooth secure simple paring and countermeasures") discloses two new Man-In-The-Middle attacks on Bluetooth secure simple paring, and security measures to render the attacks impractical.

US9463325 discloses systems and methods for maintaining a bi-directional communication link between an external device and an implantable medical device (IMD). The systems and methods receive a connection request from an external device at the IMD. The communication request includes one or more communication link parameters based on a wireless protocol. The systems and methods establish a bi-directional communication link between the external device and the IMD based on the one or more communication link parameters.

### SUMMARY

The present invention is directed to methods defined in independent claims 1 and 9, a collecting device defined in independent claim 13, a receiving device defined in independent claim 14 as well as a monitor defined in independent claim 15. Further aspects of the invention are defined in dependent claims. The disclosure mainly provides a monitor and a control method therefor, collecting and receiving devices and a communication method therefor, and is aimed to improve stability of data transmission.

According to a first aspect, an embodiment provides a method for communicating between a receiving device and a collecting device, the collecting device being configured to be connected to a patient to collect a physiological signal of the patient, and the method comprising:
acquiring, by the collecting device, configuration data from the receiving device by using a first wireless communication technology, determining first pairing information and a communication channel according to the configuration data, where the configuration data comprises a frequency parameter corresponding to the communication channel; and setting the communication channel using a second wireless communication technology according to the frequency parameter in the configuration data
communicating, by the collecting device, with the receiving device on the communication channel corresponding to the frequency parameter by using a second wireless communication technology, to acquire second pairing information that is transmitted by the receiving device on the communication channel corresponding to the frequency parameter; and
determining, by the collecting device, whether the first pairing information matches the second pairing information, and when the first pairing information matches the second pairing information, the collecting device is allowed to send to the receiving device the physiological signal or physiological information generated according to the physiological signal such that the receiving device receives the physiological signal or physiological information on the communication channel corresponding to the frequency parameter.

According to a second aspect, an embodiment provides a method for communicating between a collecting device and a receiving device, the collecting device being configured to be connected to a patient to collect a physiological signal of the patient, and the method comprising:
transmitting, by the receiving device, configuration data to the collecting device by using a first wireless communication technology, wherein the configuration data comprises a frequency parameter corresponding to a communication channel, and the frequency parameter corresponding to the communication channel in the configuration data is part of frequency parameters in communication channels supported by the collecting device; the configuration data is used to determine first pairing information and the communication channel, and further set the communication channel using a second wireless communication technology according to the frequency parameter in the configuration data; and
communicating, by the receiving device, with the collecting device on the communication channel corresponding to the frequency parameter by using a second wireless communication technology, to transmit second pairing information to the collecting device, so that after determining that the first pairing information matches the second pairing information, the collecting device is allowed to send to the receiving device the physiological signal or physiological information generated according to the physiological signal.

According to a third aspect, an embodiment provides a method for controlling a monitor, the monitor comprising at least one collecting device and a receiving device, the collecting device being configured to be connected to a patient to collect a physiological signal of the patient, and the method comprising the following steps:
transmitting, by the receiving device, configuration data to the collecting device by using a first wireless communication technology, wherein the configuration data is used to determine first pairing information and a communication channel;
acquiring, by the collecting device, the configuration data from the receiving device by using the first wireless communication technology, and determining the first pairing information and the communication channel according to the configuration data;
communicating, by the collecting device, with the receiving device on the communication channel by using a second wireless communication technology;
transmitting, by the receiving device, the second pairing information to the collecting device;
acquiring, by the collecting device, the second pairing information transmitted by the receiving device; and
determining, by the collecting device, when the first pairing information matches the second pairing information, the collecting device is allowed to send the collected physiological signal or physiological information generated according to the physiological signal to the receiving device.

According to a fourth aspect, an embodiment provides a collecting device, comprising:
a collecting unit configured to be connected to a patient to collect a physiological signal of the patient;
a first wireless unit configured to wirelessly communicate between a receiving device and using a first wireless communication technology;
a second wireless unit configured to wirelessly communicate with the receiving device by using a second wireless communication technology;
a first processing unit configured to: acquire configuration data from the receiving device by using the first wireless unit, where the configuration data comprises a frequency parameter corresponding to a communication channel; determine first pairing information and a communication channel according to the configuration data, set the communication channel using a second wireless communication technology according to the frequency parameter in the configuration data, and acquire the second pairing information that is transmitted by the receiving device on the communication channel corresponding to the frequency parameter by using the second wireless unit; and when the first processing unit determines the first pairing information matches the second pairing information, the collecting device is allowed to send the collected physiological signal or physiological information generated according to the physiological signal to the receiving device.

According to a fifth aspect, an embodiment provides a receiving device, comprising:
a third wireless unit configured to wirelessly communicate between a collecting device and using a first wireless communication technology, the collecting device being configured to be connected to a patient to collect a physiological signal of the patient;
a fourth wireless unit configured to wirelessly communicate with the collecting device by using a second wireless communication technology; and
a second processing unit configured to: transmit configuration data to the collecting device by using the third wireless unit, wherein the configuration data comprises a frequency parameter corresponding to a communication channel, and the frequency parameter corresponding to the communication channel in the configuration data is part of frequency parameters in communication channels supported by the fourth wireless unit; the configuration data is used to determine first pairing information and a communication channel, and further set the communication channel using the second wireless communication technology according to the frequency parameter in the configuration data; and communicate with the collecting device on the communication channel corresponding to the frequency parameter by using the fourth wireless unit, to transmit second pairing information to the collecting device, so that after the collecting device determines the first pairing information matches the second pairing information, the collecting device is allowed to send to the receiving device the collected physiological signal or physiological information generated according to the physiological signal.

According to a sixth aspect, an embodiment provides a monitor, comprising at least one collecting device and a receiving device, wherein
the collecting device is configured to: be connected to a patient to collect a physiological signal of the patient, acquire configuration data from the receiving device by using a first wireless communication technology, where the configuration data comprises a frequency parameter corresponding to the communication channel; determine first pairing information and a communication channel according to the configuration data, set the communication channel using the second wireless communication technology according to the frequency parameter in the configuration data; and communicate with the receiving device on the communication channel corresponding to the frequency parameter by using a second wireless communication technology such that the collecting device transmits the second pairing information on the communication channel; and when the collecting device determines the first pairing information matches the second pairing information, the collecting device is allowed to send to the receiving device the collected physiological signal or physiological information generated according to the physiological signal; and
the receiving device is configured to: transmit the configuration data to the collecting device by using the first wireless communication technology, wherein the configuration data is used to determine the first pairing information and the communication channel; and communicate with the collecting device on the communication channel corresponding to the frequency parameter by using the second wireless communication technology, to transmit the second pairing information to the collecting device.

According to a seventh aspect, an embodiment provides a monitor, comprising a memory, a processor, and a computer program stored in the memory and capable of running on the processor, wherein when executing the program, the processor implements the following steps:
acquiring configuration data from a receiving device by using a first wireless communication technology, and determining first pairing information and a communication channel according to the configuration data, the collecting device being configured to be connected to a patient to collect a physiological signal of the patient;
communicating with the receiving device on the communication channel by using a second wireless communication technology, to acquire second pairing information transmitted by the receiving device; and
determining when the first pairing information matches the second pairing information, the collecting device is allowed to send the collected physiological signal or physiological information generated according to the physiological signal to the receiving device.

According to an eighth aspect, an embodiment provides a monitor, comprising a memory, a processor, and a computer program stored in the memory and capable of running on the processor, wherein when executing the program, the processor implements the following steps:
transmitting configuration data to a collecting device by using a first wireless communication technology, wherein the configuration data is used to determine first pairing information and a communication channel, the collecting device being configured to be connected to a patient to collect a physiological signal of the patient; and
communicating with the collecting device on the communication channel by using a second wireless communication technology, to transmit second pairing information to the collecting device, so that after the collecting device determining that the first pairing information matches the second pairing information, the collecting device is allowed to send the collected physiological signal or physiological information generated according to the physiological signal to the receiving device.

According to a ninth aspect, an embodiment provides a computer-readable storage medium with a computer program stored thereon, wherein the program, when executed by a processor, implements the following steps:
acquiring configuration data from a receiving device by using a first wireless communication technology, and determining first pairing information and communication channel pairing information according to the configuration data, the collecting device being configured to be connected to a patient to collect a physiological signal of the patient;
acquiring second pairing information that is transmitted by the receiving device on the communication channel by using a second wireless communication technology; and
determining when the first pairing information matches the second pairing information, the collecting device is allowed to send the collected physiological signal or physiological information generated according to the physiological signal to the receiving device.

According to a tenth aspect, an embodiment provides a computer-readable storage medium with a computer program stored thereon, wherein the program, when executed by a processor, implements the following steps:
transmitting configuration data to a collecting device by using a first wireless communication technology, wherein the configuration data is used to determine first pairing information and a communication channel, the collecting device being configured to be connected to a patient to collect a physiological signal of the patient; and
communicating with the collecting device on the communication channel by using a second wireless communication technology, to transmit second pairing information to the collecting device, so that after the collecting device determining that the first pairing information matches the second pairing information, the collecting device is allowed to send the collected physiological signal or physiological information generated according to the physiological signal to the receiving device.

According to the monitor and the control method therefor, collecting and receiving devices and the communication method therefor in the above embodiments, configuration data is transmitted between a collecting device and a receiving device by using a first wireless communication technology, and the collecting device determines first pairing information and a communication channel according to the configuration data. Furthermore, the receiving device transmits second pairing information on the communication channel by using a second wireless communication technology, and only when the first pairing information matches the second pairing information, the collecting device is allowed to send a collected physiological signal or physiological information generated according to the physiological signal to the receiving device, so that security of wireless pairing is improved. Two wireless communication technologies are used to transmit configuration data and a control instruction or data respectively, so that signal interference is avoided, stability of data transmission is enhanced, and convenience of wireless pairing is improved due to an automatic process.

### BRIEF DESCRIPTION OF THE DRAWINGS

In order to more clearly describe technical solutions in the embodiments of the disclosure or in the prior art, the following briefly describes the accompanying drawings required for describing the embodiments or the prior art. Apparently, the accompanying drawings in the following description merely show some of the embodiments of the disclosure, and a person of ordinary skill in the art may still derive other accompanying drawings from these accompanying drawings without any creative effort.
FIG. 1 is an existing wireless measurement system for a physiological signal;
FIG. 2 is a structural block diagram of Embodiment I of a monitor provided by the disclosure;
FIG. 3 is a schematic diagram of a flow direction of a physiological signal/physiological information in a multi-parameter monitor provided by the disclosure;
FIG. 4 is a schematic diagram of a flow direction of a physiological signal/physiological information in a single-parameter monitor provided by the disclosure;
FIG. 5 is a structural block diagram of a monitor networking system composed of a monitor provided by the disclosure;
FIG. 6 is a structural block diagram of Embodiment II of a monitor provided by the disclosure;
FIG. 7 is a flowchart of a method for controlling a monitor provided by the disclosure; and
FIG. 8 is a flowchart of steps of determining, by a collecting device, first pairing information and a communication channel according to configuration data in a method for controlling a monitor provided by the disclosure.

### DESCRIPTION OF THE PREFERRED EMBODIMENTS

The disclosure will be further described in detail below through specific implementations in conjunction with the accompanying drawings. Associated similar element reference numerals are used for similar elements in different implementations. In the following implementations, many details are described such that the present application can be better understood. However, it would have been effortlessly appreciated by a person skilled in the art that some of the features could be omitted or could be substituted by other elements, materials, and methods in different cases. In certain cases, some operations involved in the present application are not displayed or described in the specification, which is to prevent a core part of the present application from being obscured by too much description. Moreover, for a person skilled in the art, the detailed description of the involved operations is not necessary, and the involved operations can be thoroughly understood according to the description in the specification and the general technical knowledge in the art.

In addition, the characteristics, operations, or features described in the specification can be combined in any appropriate manner to form various implementations. Moreover, the steps or actions in the method description can also be exchanged or adjusted in order in a way that would have been obvious to a person skilled in the art. Therefore, the various orders in the specification and the accompanying drawings are merely for the purpose of clear description of a certain embodiment and are not meant to be a necessary order unless otherwise stated that a certain order must be followed.

The serial numbers themselves for the components herein, for example, "first" and "second", are merely used to distinguish the described objects, and do not have any sequential or technical meaning. Moreover, as used in the present application, "connection" or "coupling", unless otherwise specified, comprises both direct and indirect connections (couplings).

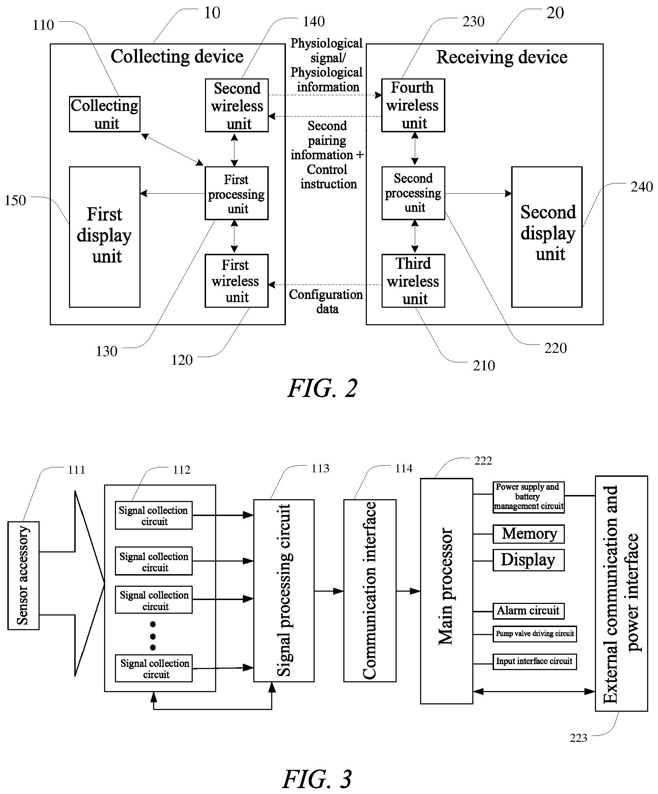

As shown in FIG. 2, a monitor provided by the disclosure comprises a receiving device 20 and at least one collecting device 10.

The collecting device 10 is configured to: be connected to a patient to collect a physiological signal of the patient, acquire configuration data from the receiving device 20 by using a first wireless communication technology, determine first pairing information and a communication channel according to the configuration data, and communicate with the receiving device 20 on the communication channel by using a second wireless communication technology, to acquire second pairing information transmitted by the receiving device 20; and determine whether the first pairing information matches the second pairing information (for example, the first pairing information and the second pairing information are the same or conform to agreed pairing rules), wherein if yes, the collecting device is allowed to send the collected physiological signal or physiological information generated according to the physiological signal to the receiving device, and if no, the collecting device is not allowed to send the collected physiological signal or the physiological information generated according to the physiological signal to the receiving device 20. That is, after determining whether the first pairing information matches the second pairing information, the collecting device 10 may transmit data (for example, the physiological signal data or the physiological information) to the receiving device 20. The first pairing information and the second pairing information may be in the form of pairing codes.

In some embodiments, the collecting device 10 is configured to: be connected to the patient to collect the physiological signal of the patient, acquire the configuration data from the receiving device 20 by using the first wireless communication technology, determine the first pairing information and the communication channel according to the configuration data, and communicate with the receiving device 20 on the communication channel by using the second wireless communication technology, to acquire a control instruction and the second pairing information transmitted by the receiving device 20; and determine whether the first pairing information matches the second pairing information (for example, the first pairing information and the second pairing information are the same or conform to agreed pairing rules), wherein if yes, the collecting device executes the control instruction and is allowed to send the collected physiological signal or the physiological information generated according to the physiological signal to the receiving device 20, wherein the first pairing information is used to uniquely identify permission to control the collecting device 10.

The collecting device 10 acquires the configuration data from the receiving device. For example, the collecting device may receive the configuration data sent by the receiving device 20, or obtain the configuration data through scanning, reading, and the like, or the receiving device 20 may write the configuration data to the collecting device 10. The collecting device 10 may obtain the configuration data through scanning. For example, the collecting device may establish wireless communication with the receiving device 20 by scanning a barcode or a two-dimensional code on the receiving device 20, or the receiving device 20 may establish wireless communication with the collecting device 10 by scanning a barcode or a two-dimensional code on the collecting device 10. The collecting device 10 may obtain the configuration data through reading, for example, obtain the configuration data by reading a radio frequency tag on the receiving device 20. Alternatively, the receiving device 20 may write the configuration data to the collecting device 10. For example, the receiving device 20 writes the configuration data to the collecting device 10 by using a radio frequency reader/writer.

In this embodiment, the collecting device 10 comprises a tag, and the receiving device 20 comprises a reader/writer. The tag comprises a first coil, and the reader/writer comprises a second coil. The second coil sends a radio frequency signal at a preset frequency, the first coil receives the radio frequency signal after entering an induction range and produces an induced current to activate the collecting device, and after being activated, the collecting device acquires the configuration data transmitted by the receiving device. That is, the reader/writer in the receiving device 20 writes the configuration data to the tag in the collecting device 10.

After acquiring the configuration data, the collecting device 10 performs configuration according to the configuration data. The first pairing information is used to uniquely identify permission to control the collecting device 10, which means that the collecting device 10 accepts the control of the receiving device 20 on the collecting device only after identifying the second pairing information that matches the first pairing information. Therefore, the control instruction and the second pairing information may not be received at the same time, that is, the collecting device 10 may first acquire the second pairing information, and then acquire the control instruction after determining that the first pairing information matches the second pairing information.

The receiving device 20 is configured to: transmit the configuration data to the collecting device 10 by using the first wireless communication technology, wherein the configuration data is used to determine the first pairing information and the communication channel; and communicate with the collecting device 10 on the communication channel by using the second wireless communication technology, to transmit the second pairing information to the collecting device 10, wherein the first pairing information is consistent with the second pairing information.

In some embodiments, the receiving device 20 is configured to: transmit the configuration data to the collecting device 10 by using the first wireless communication technology, wherein the configuration data is used to determine the first pairing information and the communication channel; and communicate with the collecting device 10 on the communication channel by using the second wireless communication technology, to transmit the control instruction and the second pairing information to the collecting device 10, wherein the first pairing information is consistent with the second pairing information.

The consistency described herein means that the second pairing information transmitted by the receiving device 20 to the collecting device 10 is the same as the first pairing information in the configuration data, or there is a pairing relationship between the second pairing information and the first pairing information (for example, conforming to agreed pairing rules). After the pairing succeeds, the first pairing information may not always be valid, and may be invalid under preset conditions (for example, an active condition and a passive condition). For example, after an instruction to modify the first matching code or an indication that the first matching code is invalid that is transmitted by the receiving device 20 is acquired, the first matching code becomes invalid. For another example, the first matching code becomes invalid within a preset duration after the pairing succeeds or within a preset duration after the collecting device 10 receives data or an instruction from the receiving device 20 for the last time.

It can be seen that in the wireless pairing between the collecting device 10 and the receiving device 20, only when the first pairing information matches the second pairing information, the collecting device 10 establishes a communication connection with the receiving device 20, for example, transmits the collected physiological signal data or the physiological information mentioned below to the receiving device 20, or executes the control instruction sent by the receiving device 20, so that reliability and security of the wireless pairing are improved. Two wireless communication technologies are used to transmit configuration data and a control instruction or data respectively, so that signal interference is avoided, stability of data transmission is enhanced, and convenience of wireless pairing is improved due to an automatic process.

The collecting device 10 may comprise: a collecting unit 110, a first wireless unit 120, a first processing unit 130, a second wireless unit 140, and a first display unit 150. The collecting unit 110, the first wireless unit 120, the second wireless unit 140, and the first display unit 150 are all communicatively connected to the first processing unit 130.

The collecting unit 110 is configured to be connected to a patient to collect a physiological signal of the patient, the physiological signal being a signal directly measured by the collecting unit 110. For example, at least one of physiological signals such as electrocardiogram (ECG), oxygen saturation (SpO2), temperature (TEMP), non-invasive blood pressure (NIBP), respiratory rate (RESP), and invasive blood pressure (IBP) of the patient may be collected.

The first wireless unit 120 is configured to wirelessly communicate with the receiving device 20 by using the first wireless communication technology. The first wireless communication technology may comprise a radio frequency identification (RFID) technology or a near field communication (NFC) technology. Certainly, the first wireless communication technology may further be a wireless local area network technology. The collecting device 10 may establish wireless communication with the receiving device 20 by scanning the barcode or the two-dimensional code on the receiving device 20, or the receiving device 20 may establish wireless communication with the collecting device 10 by scanning the barcode or the two-dimensional code on the collecting device 10. In the following description, the NFC technology is mainly used as an example.

The second wireless unit 140 is configured to wirelessly communicate with the receiving device 20 by using the second wireless communication technology. The second wireless communication technology may comprise at least one of a wireless medical telemetry service (WMTS) technology, a wireless local area network technology, a Bluetooth communication technology, and a ZigBee communication technology. It can be seen that the first wireless unit 120 and the second wireless unit 140 use different wireless communication technologies to isolate transmission of the configuration data and transmission of the control instruction, so that the security and stability of wireless pairing and subsequent data transmission are improved.

In some embodiments, the first wireless communication technology and the second wireless communication technology may belong to a same wireless communication technology, which is not limited herein.

The first processing unit 130 is configured to: acquire the configuration data from the receiving device 20 by using the first wireless unit 120, determine the first pairing information and the communication channel according to the configuration data, and communicate with the receiving device 20 on the communication channel by using the second wireless unit 140, to acquire the second pairing information transmitted by the receiving device 20; and determine whether the first pairing information matches the second pairing information, if yes, establish a communication connection with the receiving device 20, and if no, skip establishing the communication connection with the receiving device 20.

In some embodiments, the first processing unit 130 is configured to: acquire the configuration data from the receiving device 20 by using the first wireless unit 120, determine the first pairing information and the communication channel according to the configuration data, and communicate with the receiving device 20 on the communication channel by using the second wireless unit 140, to acquire the control instruction and the second pairing information transmitted by the receiving device 20; and determine whether the first pairing information matches the second pairing information, wherein if yes, the collecting device executes the control instruction and is allowed to send the collected physiological signal or the physiological information generated according to the physiological signal to the receiving device 20, for example, transmit the collected physiological signal data or the physiological information to the receiving device 20, wherein the first pairing information is used to uniquely identify permission to control the collecting device 10.

Since the physiological signal is measurement data (physiological signal data) directly collected from the patient and needs to be processed, the first processing unit 130 may process the physiological signal to obtain the physiological information, or the physiological signal may be sent to the receiving device 20, and the receiving device 20 processes the physiological signal to obtain the physiological information. The physiological information may comprise at least one of ECG information, SpO2 information, TEMP information, NIBP information, RESP information, and IBP information. The control instruction is an instruction for controlling the collecting device, for example, may comprise an instruction for controlling the collecting device 10 to send the physiological signal or the physiological information, or may be used for setting a collecting parameter (for example, a collecting time, a collecting duration, and a collecting item) of the collecting device 10. said executing, by the first processing unit 130, the control instruction may comprise: transmitting, by using the second wireless unit 140, the collected physiological signal or the physiological information generated according to the physiological signal to the receiving device 20.

The first processing unit 130 may further be configured to discard or ignore subsequent data sent by the receiving device, and prohibit the first wireless unit 120 from sending the physiological signal or the physiological information to the outside on the configured channel, when determining that the first pairing information does not match the second pairing information. Discarding subsequent data means discarding subsequent data that has been received, and ignoring subsequent data means refusing to receive or not receiving subsequent data. In this way, it may be ensured that after the receiving device 20 is paired with and connected to a new collecting device 10 again, the previously paired collecting device 10 does not send data on a same channel, thereby avoiding interference to the reliability of communication between the new paired devices.

The first display unit 150 may be configured to display a human-machine interaction interface, a physiological signal, or physiological information generated according to the physiological signal, for example, display the physiological signal or the physiological information in the form of text, charts, waveforms, and the like, so that a doctor can understand physiological information of a patient. The first display unit 150 may be a touch display screen, which can not only perform human-machine interaction but also display various types of data and information.

The receiving device 20 may comprise: a third wireless unit 210, a second processing unit 220, a fourth wireless unit 230, and a second display unit 240. The third wireless unit 210, the fourth wireless unit 230, and the second display unit 240 are all communicatively connected to the second processing unit 220.

The third wireless unit 210 is configured to wirelessly communicate with the first wireless unit 120 of the collecting device 10 by using the first wireless communication technology.

The fourth wireless unit 230 is configured to wirelessly communicate with the second wireless unit 140 of the collecting device 10 by using the second wireless communication technology.

The second processing unit 220 is configured to: transmit the configuration data to the first wireless unit 120 by using the third wireless unit 210; and communicate with the second wireless unit 140 on the communication channel by using the fourth wireless unit 230, to transmit the second pairing information to the second wireless unit 140, so that after determining that the first pairing information matches the second pairing information, the first processing unit 130 is allowed to send the collected physiological signal or the physiological information generated according to the physiological signal to the receiving device 20. In some embodiments, the second processing unit 220 is configured to: transmit the configuration data to the first wireless unit 120 by using the third wireless unit 210; and communicate with the second wireless unit 140 on the communication channel by using the fourth wireless unit 230, to transmit the control instruction and the second pairing information to the second wireless unit 140, so that after determining that the first pairing information matches the second pairing information, the first processing unit 130 executes the control instruction (for example, transmitting collected physiological signal data or the physiological information to the receiving device 20) and is allowed to send the collected physiological signal or the physiological information generated according to the physiological signal to the receiving device 20.

The second display unit 240 may be configured to display a human-machine interaction interface and the physiological signal or the physiological information received by the third wireless unit 210. The second display unit 240 may be a touch display screen, which can not only perform human-machine interaction but also display various types of data and information.

The monitor provided by the disclosure may be used in a ward, and the collecting device 10 may be a portable collecting device. For a scene in which a plurality of patients in the ward need to be monitored at the same time, pairing connections between a plurality of collecting devices 10 and the receiving device 20 according to the above process may be conveniently arranged one by one, so that the medical personnel can conveniently complete the pairing connection between the collecting device 10 and the receiving device 20. In addition, it may be ensured that when a plurality of collecting devices 10 are used at the same time, a pairing relationship between each collecting device 10 and the receiving device 20 is not damaged, and data communication is not interfered, so that integrity of collected physiological signals or physiological information is ensured.

The configuration data may comprise: a frequency parameter corresponding to the communication channel, patient information of the patient, the first pairing information, or a receiving device identification code used to generate the first pairing information. The receiving device identification code is an identification code for identifying the receiving device, and may be, for example, an apparatus code, a hardware address such as Medium/Media Access Control (MAC), and the like of the receiving device. The patient information of the patient may be input by the medical personnel on the receiving device 20. The wireless interaction between the collecting device 10 and the receiving device 20 may specifically be performed in a plurality of manners, so that the configuration data may comprise the first pairing information or may not comprise the first pairing information, which will be further described by using several embodiments below.

### Embodiment I:

In this embodiment, the configuration data may comprise: the frequency parameter corresponding to the communication channel, the patient information of the patient, and the first pairing information. When the monitor is in use, the patient information is input by the medical personnel on the second display unit 240, the first pairing information is generated by the second processing unit 220, and the frequency parameter corresponding to the communication channel may be a frequency parameter and a frequency band parameter, and may be preset by the second processing unit 220. In this embodiment, the frequency parameter corresponding to the communication channel in the configuration data may be only some of frequency parameters in communication channels supported by the fourth wireless unit 230, and the fourth wireless unit 230 may wirelessly communicate with a plurality of collecting devices 10 on different channels without mutual influence. That is, the receiving device 20 may prestore a plurality of frequency parameters, and each frequency parameter corresponds to a communication channel, so that the receiving device 20 can wirelessly communicate with different collecting devices 10 on different communication channels.

The second processing unit 220 may generate the first pairing information in a plurality of manners, provided that the generated first pairing information being unique is ensured. For example, in this embodiment, the second processing unit 220 generates the first pairing information according to information about a number of times pairing has been performed. The information about the number of times pairing has been performed may be a number of times the receiving device 20 has been paired with or successfully paired with the collecting device (which is not limited to the current collecting device). Therefore, the information about the number of times pairing has been performed is recorded and updated each time the receiving device 20 is successfully paired with a collecting device 10. For another example, the second processing unit 220 generates the first pairing information according to current time information (for example, accurate to a day or an hour or a minute). The first pairing information may be 8-bit, 12-bit, or 16-bit data, and the first pairing information generated each time is unique.

Before or after the medical personnel connect the collecting device 10 to the patient, the collecting device 10 may be aimed at and close to a designated area (for example, an area corresponding to an NFC reader/writer) of the receiving device 20. In this case, the first wireless unit 120 acquires, by using the first wireless communication technology, the configuration data stored in the third wireless unit 210, the first wireless unit 120 comprises a tag, and the third wireless unit 210 comprises a reader/writer. In this embodiment, the reader/writer is a radio frequency reader/writer, and the tag is a radio frequency tag. The tag comprises a first coil, and the reader/writer comprises a second coil. The second coil sends a radio frequency signal at a preset frequency, the first coil receives the radio frequency signal after entering an induction range and produces an induced current to activate the collecting device 10, and after being activated, the collecting device 10 acquires the configuration data transmitted by the receiving device 20. The medical personnel only need to aim the radio frequency reader/writer at the radio frequency tag, and then the radio frequency reader/writer can write the configuration data to the radio frequency tag, which is very convenient and forms one-to-one communication, so that transmission of the configuration data is secure and reliable and is not prone to interference. Certainly, in other embodiments, the first wireless unit 120 may comprise a reader/writer, and the third wireless unit 210 comprises a tag, which is not limited herein.

After the configuration data is written to the first wireless unit 120, the first processing unit 130 determines the first pairing information according to the first pairing information in the configuration data, and determines the communication channel according to the frequency parameter corresponding to the communication channel. The frequency parameter comprises a frequency band, a frequency, and/or the like.

The first processing unit 130 sets the communication channel of the second wireless unit 140 according to the frequency parameter in the configuration data, so that the second wireless unit 140 communicates with the fourth wireless unit 230 on the communication channel corresponding to the same frequency band or frequency. In addition, the first processing unit may display the read patient information on the first display unit 150, which is convenient for the medical personnel and the patient to confirm; and store the read first pairing information. In this embodiment, the second wireless unit 140 and the fourth wireless unit 230 perform wireless communication on the communication channel corresponding to the same frequency band or frequency, which is implemented by using the wireless medical telemetry service technology. The use of such a telemetry technology makes small changes to the existing monitor and has a wide range of application.

After the receiving device 20 completes the above pairing, the second processing unit 220 may control the second display unit 240 to display a paired state, enable the fourth wireless unit 230 to send the second pairing information to the outside on the frequency band or the frequency, and further send the control instruction in some embodiments.

The second wireless unit 140 receives the second pairing information on the frequency band or the frequency set by the first processing unit 130, and outputs the second pairing information to the first processing unit 130. The first processing unit 130 compares the second pairing information with the stored first pairing information. If the second pairing information matches the stored first pairing information (for example, the second pairing information and the first pairing information are the same or conform to agreed matching rules), the first processing unit is allowed to send the collected physiological signal or the physiological information generated according to the physiological signal to the receiving device 20, and if the second pairing information does not match the first pairing information, the first processing unit is not allowed to send the collected physiological signal or the physiological information generated according to the physiological signal to the receiving device 20. In some embodiments, the second wireless unit 140 receives the second pairing information and the control instruction on the frequency band or the frequency set by the first processing unit 130, and outputs the second pairing information and the control instruction to the first processing unit 130. The first processing unit 130 compares the second pairing information with the stored first pairing information. If the second pairing information matches the stored first pairing information (for example, the second pairing information and the first pairing information are the same or conform to agreed matching rules), the first processing unit executes the control instruction (for example, performing corresponding setting on the collecting unit 110 according to the control instruction, or sending the physiological signal or the physiological information to the receiving device 20 by using the second wireless unit 140) and is allowed to send the collected physiological signal or the physiological information generated according to the physiological signal to the receiving device 20. If the second pairing information does not match (for example, is different from) the stored first pairing information, the first processing unit discards or ignores subsequent data sent by the receiving device, and/or is prohibited from sending the collected physiological signal or the physiological information generated according to the physiological signal to the receiving device 20.

The fourth wireless unit 230 receives data such as the physiological signal or the physiological information on the communication channel on the corresponding frequency band or frequency, and outputs the data to the second processing unit 220. The second processing unit 220 displays, by using the second display unit 240, the received apparatus state information of the collecting device, waveforms and parameters corresponding to the physiological signal or the physiological information of the patient, and the like.

The monitor may be a multi-parameter monitor or a single-parameter monitor. The multi-parameter monitor is used as an example. A transmission process of a physiological signal/physiological information of the multi-parameter monitor is shown in FIG. 3. For example, the second wireless unit 140 outputs the physiological information. The collecting unit 110 comprises a physiological parameter sensor accessory 111, a signal collection circuit 112, and a signal processing circuit 113. The collecting device 10 is a portable monitoring apparatus with an independent housing, and a sensor interface area is arranged on a housing panel. A plurality of sensor interfaces are integrated in the sensor interface area and configured to be connected to various external physiological parameter sensor accessories 111. The second processing unit 220 comprises a main processor 222 and an external communication and power interface 223. A housing panel of the receiving device 20 comprises a small IXD display area, a display, an input interface circuit, an alarm circuit (such as an LED alarm area), and the like. The external communication and power interface 223 is configured to be connected to a communication module to communicate with a host and obtain power from the outside. The signal collection circuit 112 of the portable monitoring apparatus may be selected from an electrocardiogram circuit, a respiration circuit, a body temperature circuit, a blood oxygen circuit, a non-invasive blood pressure circuit, an invasive blood pressure circuit, and the like. These signal collection circuits 112 are respectively electrically connected to corresponding sensor interfaces, so as to be electrically connected to sensor accessories 111 corresponding to different physiological parameters. An output end of the signal collection circuit is coupled to a front-end signal processor (the signal processing circuit 113), and the front-end signal processor is electrically connected to the second wireless unit 140 by using a communication interface 114. The fourth wireless unit 230 is connected to the main processor 222, and the main processor 222 is electrically connected to the external communication and power interface 223. The external communication and power interface 223 is communicatively connected to an external host, a central station, and the like. Various physiological parameter measurement circuits can use common circuits in the prior art. The front-end signal processor completes sampling and analog-to-digital conversion of an output signal of the signal collection circuit, and outputs a control signal to control a measurement process of the physiological signal. These parameters comprise but are not limited to: parameters such as electrocardiogram, respiration, body temperature, blood oxygen, non-invasive blood pressure, and invasive blood pressure. The front-end signal processor may be implemented by using a single-chip microcomputer or other semiconductor devices, for example, by using a single-chip microcomputer, an ASIC, or an FPGA. After being simply processed and packaged, data obtained through sampling by the front-end signal processor is sent to the main processor 222 by using the second wireless unit 140. Wireless communication is performed between the collecting device 10 and the receiving device 20, which can also play a role in isolating the patient from a power supply apparatus. The main processor 222 completes calculation of the physiological information, and sends a calculation result and waveforms of the physiological information to the host (such as a host with a display, a PC, and a central station) through the external communication and power interface 223. The external communication and power interface 223 may be one of local area network interfaces composed of the Ethernet, a token ring, a token bus, and a fiber distributed data interface (FDDI) as the backbone network of these three networks, or a combination thereof, or may be one of wireless interfaces such as an infrared interface, a Bluetooth interface, a Wi-Fi interface, and a WMTS communication interface, or a combination thereof, or may be one of wired data connection interfaces such as an RS232 interface and a USB interface, or a combination thereof. The external communication and power interface 223 may also be one of a wireless data transmission interface and a wired data transmission interface or a combination thereof. The host may be any computer apparatus such as a host of a monitor, an electrocardiograph, an ultrasonic diagnostic apparatus, and a computer, and after being installed with matching software, the host can form a monitoring apparatus. The host may further be a communication apparatus such as a mobile phone, and the collecting device sends, by using a Bluetooth interface, data to the mobile phone supporting Bluetooth communication, so as to implement remote transmission of the data.

If the second wireless unit 140 outputs the physiological signal, the collecting unit 110 comprises the physiological parameter sensor accessory 111 and the signal collection circuit 112. The signal processing circuit 113 is arranged in the receiving device 20, and a connection relationship between other functional modules remains unchanged.

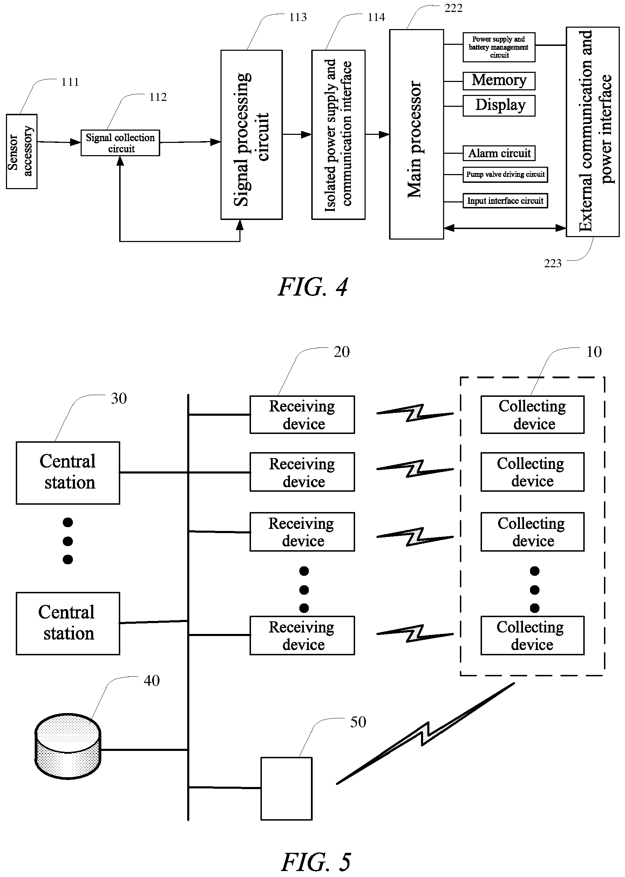

As shown in FIG. 4, an architecture of a monitor with a single physiological parameter is provided. For the same content, reference may be made to the above content.

FIG. 5 shows a monitor networking system used in a hospital. By using the system, data of the monitor may be saved as a whole to centrally manage patient information and nursing information that are stored in association, which facilitates storage of historical data and alarming in association. In the system shown in FIG. 5, a bedside monitor, namely, a receiving device 20, may be provided for each hospital bed. In addition, each receiving device 20 may further be paired with one or more portable monitoring apparatuses, namely, collecting devices 10, for transmission. The collecting device 10 provides a simple and portable parameter processing module, and the simple and portable parameter processing module may be worn on the body of a patient to perform mobile monitoring for the patient. After the collecting device 10 and the receiving device 20 perform wireless communication, a physiological signal/physiological information generated through mobile monitoring may be transmitted to the receiving device 20 for display, or transmitted, by using the receiving device 20, to a central station 30 for a doctor or a nurse to check, or transmitted to a data server 40 for storage by using the receiving device 20. In addition, the collecting device 10 may further directly transmit, by using a wireless network node 50 arranged in the hospital, the physiological signal/physiological information generated through mobile monitoring to the central station 30 for storage and display, or transmit, by using the wireless network node 50 arranged in the hospital, the physiological signal/physiological information generated through mobile monitoring to the data server 40 for storage. It can be seen that data corresponding to a physiological parameter displayed on the receiving device 20 may originate from the collecting device 10 or from the data server. The collecting device 10 may be connected to a sensor accessory 111 in a wired and/or wireless manner, and comprise a part or all of the circuits of the above parameter processing module. For example, isolation measures for isolating patients may not be provided in the collecting device 10 but provided outside the collecting device 10, for example, provided on the sensor accessory 111. The collecting device 10 may be equipped with a display screen for displaying a parameter calculation result and/or alarm prompt information. For example, the collecting device 10 may be a wireless sensor patch attached to the body, or a transfer monitor, or a telemetry apparatus.

### Embodiment II:

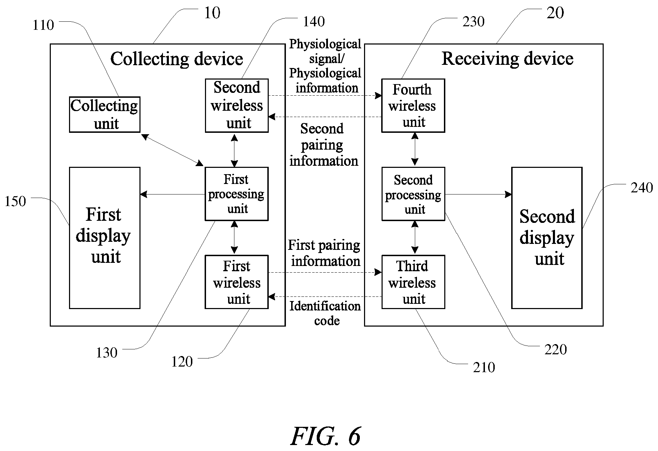

Referring to FIG. 6, a difference between this embodiment and the previous embodiment lies in that: the first pairing information is generated and transmitted in different manners. The configuration data may comprise the patient information of the patient, the receiving device identification code used to generate the first pairing information, and the frequency parameter corresponding to the communication channel. said determining, by the first processing unit 130, the first pairing information and the communication channel according to the configuration data comprises: generating, by the first processing unit 130, the first pairing information according to the receiving device identification code, and determining the communication channel according to the frequency parameter corresponding to the communication channel; and transmitting the first pairing information to the third wireless unit 210 by using the first wireless unit 120.
said generating, by the first processing unit 130, the first pairing information according to the receiving device identification code may comprise: calculating, by the first processing unit 130, the first pairing information according to the receiving device identification code and a collecting device identification code of the collecting device 10 by using a preset algorithm, so that the first pairing information comprises identification information of both the collecting device 10 and the receiving device 20, thereby reflecting a pairing relationship between the devices. In this way, it is difficult for a third party to learn of the first pairing information, so that the security is improved during matching of the subsequent second pairing information and the first pairing information. The receiving device identification code and the collecting device identification code are used to uniquely identify the receiving device and the collecting device respectively, for example, an ID number of the device itself, and a number of the device in the hospital. Certainly, the first processing unit 130 may generate the first pairing information according to the receiving device identification code in other manners. For example, the first processing unit 130 calculates the first pairing information according to the receiving device identification code by using a specific preset algorithm of the collecting device itself, that is, different collecting devices apply different preset algorithms. Since transmission of the subsequent second pairing information and the control instruction and matching of the second pairing information and the first pairing information are the same as those in the previous embodiment, details are not described again.

It can be seen that in the monitor provided by the disclosure, a collecting device 10 may be worn on the body of a patient to implement collection and measurement of various physiological signals of the patient, and send the collected and measured physiological signals or physiological information to a receiving device 20 in a wireless manner, wherein the receiving device 20 may be a bedside monitor or a remote central station. Due to the wireless matching and safe and reliable communication between the collecting device 10 and the receiving device 20, a plurality of collecting devices 10 may be connected to the same receiving device 20 at different time points without mutual influence. When the patient is monitored by using the monitor, the patient will not be restricted to the hospital bed, and an activity range of the patient may be increased, which facilitate the recovery of the patient.

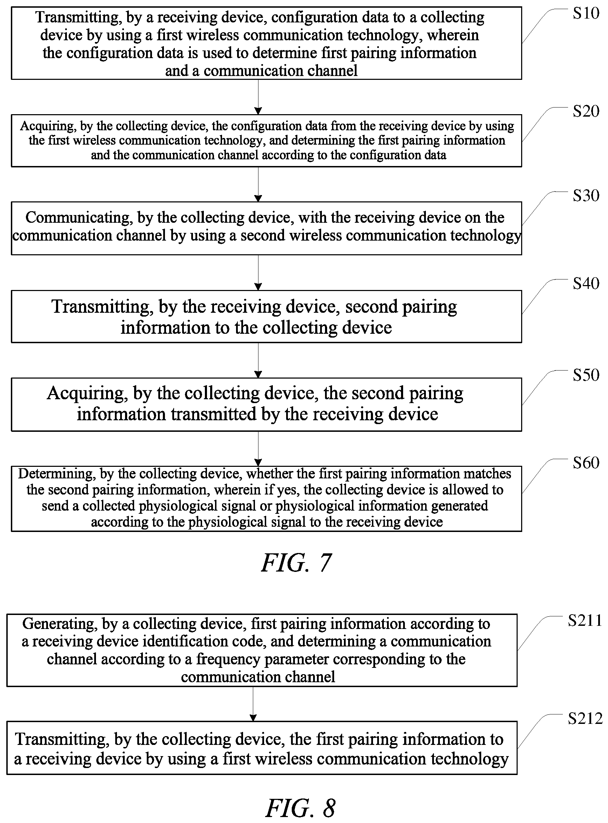

Based on the monitor provided in the above embodiment, the disclosure further provides a corresponding method for controlling a monitor. As shown in FIG. 7, the method comprises the following steps S10 to S60.

Step S10, transmitting, by the above receiving device, configuration data to the above collecting device by using a first wireless communication technology, wherein the configuration data is used to determine first pairing information and a communication channel, the first pairing information being used to uniquely identify permission to control the collecting device. The configuration data comprises: patient information of a patient, the first pairing information, or a receiving device identification code used to generate the first pairing information, and a frequency parameter corresponding to the communication channel. The collecting device is configured to be connected to a patient to collect a physiological signal of the patient.

Step S20, acquiring, by the collecting device, the configuration data from the receiving device by using the first wireless communication technology, and determining the first pairing information and the communication channel according to the configuration data. The first wireless communication technology may comprise a radio frequency identification (RFID) technology or a near field communication (NFC) technology. Certainly, the first wireless communication technology may further be a wireless local area network technology.

Step S30, communicating, by the collecting device, with the receiving device on the communication channel by using a second wireless communication technology. The second wireless communication technology comprises at least one of a wireless medical telemetry service technology, a wireless local area network technology, a Bluetooth communication technology, and a ZigBee communication technology. In some embodiments, the first wireless communication technology and the second wireless communication technology may belong to a same wireless communication technology, which is not limited herein.

Step S40, transmitting, by the receiving device, second pairing information to the collecting device. In some embodiments, the receiving device transmits the second pairing information to the collecting device.

Step S50, acquiring, by the collecting device, the second pairing information transmitted by the receiving device. In some embodiments, the collecting device acquires a control instruction and the second pairing information transmitted by the receiving device.

Step S60, determining, by the collecting device, whether the first pairing information matches the second pairing information, if yes, establishing a communication connection with the receiving device, and if no, skipping establishing the communication connection with the receiving device. In some embodiments, the collecting device determines whether the first pairing information matches the second pairing information, wherein if yes, the collecting device executes the control instruction and is allowed to send the collected physiological signal or physiological information generated according to the physiological signal to the receiving device, and if no, the collecting device discards or ignores subsequent data sent by the receiving device, and/or is prohibited from sending the collected physiological signal or the physiological information generated according to the physiological signal to the receiving device.

The configuration data may comprise the first pairing information and the frequency parameter corresponding to the communication channel, the first pairing information being generated by the receiving device. For example, the first pairing information is generated by the receiving device according to information about a number of times pairing has been performed.

Alternatively, the configuration data may comprise the receiving device identification code used to generate the first pairing information, and the frequency parameter corresponding to the communication channel, and the first pairing information is generated by the collecting device according to the receiving device identification code.

The collecting device comprises a tag, and the receiving device comprises a reader/writer. The tag comprises a first coil, and the reader/writer comprises a second coil. The second coil sends a radio frequency signal at a preset frequency, the first coil receives the radio frequency signal after entering an induction range and produces an induced current to activate the collecting device, and after being activated, the collecting device acquires the configuration data transmitted by the receiving device. Certainly, in other embodiments, the collecting device may comprise a reader/writer, and the receiving device comprises a tag, which is not limited herein.

The method involves the interaction between the collecting device and the receiving device, and therefore comprises a method for communicating with the receiving device by the collecting device, and a method for communicating with the collecting device by the receiving device to a. Specifically, the method for communicating with the receiving device by the collecting device comprises step S20, step S30, step S50, and step S60.

Step S20, acquiring, by the collecting device, the configuration data from the receiving device by using the first wireless communication technology, and determining the first pairing information and the communication channel according to the configuration data. The collecting device is configured to be connected to a patient to collect a physiological signal of the patient. The configuration data is used to determine the first pairing information and the communication channel, the first pairing information being used to uniquely identify permission to control the collecting device. The configuration data comprises: patient information of a patient, the first pairing information, or a receiving device identification code used to generate the first pairing information, and a frequency parameter corresponding to the communication channel. The collecting device acquires the configuration data from the receiving device. Specifically, the collecting device may receive the configuration data sent by the receiving device, or obtain the configuration data through scanning, reading, and the like. The first wireless communication technology may comprise a radio frequency identification technology or a near field communication technology. The collecting device comprises a tag, and the receiving device comprises a reader/writer. The tag comprises a first coil, and the reader/writer comprises a second coil. The second coil sends a radio frequency signal at a preset frequency, the first coil receives the radio frequency signal after entering an induction range and produces an induced current to activate the collecting device, and after being activated, the collecting device acquires the configuration data transmitted by the receiving device. Certainly, in other embodiments, the collecting device may comprise a tag, and the receiving device comprises a reader/writer.

Likewise, the first pairing information may be generated by the receiving device or the collecting device.

For example, the first pairing information is generated by the receiving device. The configuration data comprises the patient information of the patient, the first pairing information, and the frequency parameter corresponding to the communication channel. The step of determining, by the collecting device, the first pairing information and the communication channel according to the configuration data comprises: determining, by the collecting device, the first pairing information according to the first pairing information in the configuration data, and determining the communication channel according to the frequency parameter corresponding to the communication channel.

For example, the first pairing information is generated by the collecting device. The configuration data comprises the patient information of the patient, the receiving device identification code used to generate the first pairing information, and the frequency parameter corresponding to the communication channel. As shown in FIG. 8, the step of determining, by the collecting device, the first pairing information and the communication channel according to the configuration data comprises the following substeps S211 and S212.

Step S211, generating, by the collecting device, the first pairing information according to the receiving device identification code, and determining the communication channel according to the frequency parameter corresponding to the communication channel; and
Step S212, transmitting, by the collecting device, the first pairing information to the receiving device by using the first wireless communication technology.

Step S30, communicating, by the collecting device, with the receiving device on the communication channel by using a second wireless communication technology.

Step S50, acquiring, by the collecting device, the second pairing information transmitted by the receiving device. In some embodiments, the collecting device acquires a control instruction and the second pairing information transmitted by the receiving device.

Step S60, determining, by the collecting device, whether the first pairing information matches the second pairing information, wherein if yes, the collecting device is allowed to send the collected physiological signal or physiological information generated according to the physiological signal to the receiving device; and if no, the collecting device discards or ignores subsequent data sent by the receiving device, and/or is prohibited from sending the collected physiological signal or the physiological information generated according to the physiological signal to the receiving device. In some embodiments, the collecting device determines whether the first pairing information matches the second pairing information, and if yes, sends the collected physiological signal or the physiological information generated according to the physiological signal to the receiving device.

The method for communicating with the collecting device by the receiving device comprises the following steps S10 and S40.

Step S10, transmitting, by the receiving device, configuration data to the collecting device by using a first wireless communication technology. The configuration data is used to determine first pairing information and a communication channel, the first pairing information being used to uniquely identify permission to control the collecting device. The configuration data comprises: patient information of a patient, the first pairing information, or a receiving device identification code used to generate the first pairing information, and a frequency parameter corresponding to the communication channel. The collecting device is configured to be connected to a patient to collect a physiological signal of the patient. The receiving device transmits the configuration data to the collecting device, for example, may send the configuration data to the collecting device, or write the data to the collecting device, or the collecting device may obtain the configuration data by scanning and reading the receiving device. The first wireless communication technology may comprise a radio frequency identification (RFID) technology or a near field communication (NFC) technology. Certainly, the first wireless communication technology may further be a wireless local area network technology.

Step S40, communicating, by the receiving device, with the collecting device on the communication channel by using the second wireless communication technology, to transmit second pairing information to the collecting device, so that after determining that the first pairing information matches the second pairing information, the collecting device is allowed to send the collected physiological signal or physiological information generated according to the physiological signal to the receiving device, wherein the first pairing information is consistent with the second pairing information.

In some embodiments, the receiving device communicates with the collecting device on the communication channel by using the second wireless communication technology, to transmit a control instruction and the second pairing information to the collecting device, so that after determining that the first pairing information matches the second pairing information, the collecting device executes the control instruction and is allowed to send the collected physiological signal or the physiological information generated according to the physiological signal to the receiving device, wherein the first pairing information is consistent with the second pairing information.

The second wireless communication technology comprises at least one of a wireless medical telemetry service technology, a wireless local area network technology, a Bluetooth communication technology, and a ZigBee communication technology. In some embodiments, the first wireless communication technology and the second wireless communication technology may belong to a same wireless communication technology, which is not limited herein.

The configuration data may comprise the first pairing information and the frequency parameter corresponding to the communication channel, the first pairing information being generated by the receiving device. For example, the first pairing information is generated by the receiving device according to information about a number of times pairing has been performed.

Alternatively, the configuration data may comprise the receiving device identification code used to generate the first pairing information, and the frequency parameter corresponding to the communication channel, and the first pairing information is generated by the collecting device according to the receiving device identification code.

The collecting device comprises a tag, and the receiving device comprises a reader/writer. The tag comprises a first coil, and the reader/writer comprises a second coil. The second coil sends a radio frequency signal at a preset frequency, the first coil receives the radio frequency signal after entering an induction range and produces an induced current to activate the collecting device, and after being activated, the collecting device acquires the configuration data transmitted by the receiving device. Certainly, in other embodiments, the collecting device may comprise a reader/writer, and the receiving device comprises a tag, which is not limited herein.

Since specific details, principles, and effects of the control method have been described in detail in the above embodiments of the monitor, details are not described herein again.

A person skilled in the art may understand that all or some of the steps of the various methods in the above implementations may be completed by a program instructing related hardware. The program may be stored in a computer-readable storage medium, and the storage medium may comprise: a read-only memory, a random access memory, a magnetic disk, an optical disk, or the like. For example, a program corresponding to the method for communicating with the receiving device by the collecting device is stored in a memory of the collecting device, and when wireless pairing and interaction need to be performed, the steps in the method for communicating with the receiving device by the collecting device can be implemented by executing the program in the memory by using a processor. A program corresponding to the method for communicating with the collecting device by the receiving device is stored in a memory of the receiving device, and when wireless pairing and interaction need to be performed, the steps in the method for communicating with the collecting device by the receiving device can be implemented by executing the program in the memory by using a processor. Especially, in the actual implementation process of the disclosure, the steps in the above embodiments may also be written as independent programs. The program may be stored on a server, a magnetic disk, an optical disk, and a flash disk and can be downloaded and saved in a memory of a local apparatus, or the local system can be updated through downloading. The above functions can be implemented by executing the program in the memory by using a processor.

The disclosure has been described with reference to specific examples, which are merely for the purpose of facilitating understanding of the disclosure and are not intended to limit the disclosure. For a person skilled in the technical field to which the disclosure belongs, several simple deductions, variations, or replacements may also be made according to the idea of the disclosure.

## Claims

1. A method for communicating between a receiving device (20) and a collecting device (10), the collecting device (10) is configured to be connected to a patient to collect a physiological signal of the patient, and the method comprises:
acquiring, by the collecting device (10), configuration data from the receiving device (20) by using a first wireless communication technology, and determining first pairing information according to the configuration data, wherein the configuration data comprises a frequency parameter corresponding to a communication channel;
communicating, by the collecting device (10), with the receiving device (20) by using a second wireless communication technology, to acquire second pairing information transmitted by the receiving device (20); and
determining, by the collecting device (10), whether the first pairing information matches the second pairing information, and when the first pairing information matches the second pairing information, the collecting device (10) is allowed to send to the receiving device (20) the physiological signal or physiological information generated according to the physiological signal such that the receiving device (20) receives the physiological signal or physiological information on the communication channel corresponding to the frequency parameter;
wherein the method, executed by the collecting device (10), further comprises:
determining the communication channel according to the configuration data, setting the communication channel using the second wireless communication technology according to the frequency parameter in the configuration data, acquiring the second pairing information that is transmitted by the receiving device (20) on the communication channel corresponding to the frequency parameter.

2. The method of claim 1, **characterized in that** the first pairing information is used to uniquely identify a control authority of the collecting device (10); the method further comprises:
communicating, by the collecting device (10), with the receiving device (20) on the communication channel by using the second wireless communication technology, to acquire a control instruction transmitted by the receiving device (20); and
when the collecting device (10) determines that the first pairing information matches the second pairing information, executing, by the collecting device (10), the control instruction

3. The method of claim 1, **characterized in that** the configuration data comprises the first pairing information and the frequency parameter corresponding to the communication channel, the first pairing information being generated by the receiving device (20); the frequency parameter corresponding to the communication channel in the configuration data is part of frequency parameters in communication channels supported by the collecting device (10), so that the receiving device (20) is capable of wirelessly communicating with different collecting devices (10) on different communication channels; and
determining, by the collecting device (10), first pairing information and a communication channel according to the configuration data comprises:
determining, by the collecting device (10), the first pairing information according to the first pairing information in the configuration data, and determining the communication channel according to the frequency parameter corresponding to the communication channel.

4. The method of claim 1, **characterized in that** the configuration data comprises a receiving device identification code used to generate the first pairing information, and the frequency parameter corresponding to the communication channel; the frequency parameter corresponding to the communication channel in the configuration data is part of frequency parameters in communication channels supported by the collecting device (10), so that the receiving device (20) is capable of wirelessly communicating with different collecting devices (10) on different communication channels; and
determining, by the collecting device (10), first pairing information and a communication channel according to the configuration data comprises:
generating, by the collecting device (10), the first pairing information according to the receiving device identification code, and determining the communication channel according to the frequency parameter corresponding to the communication channel; and the method further comprises:
transmitting, by the collecting device (10), the first pairing information to the receiving device (20) by using the first wireless communication technology.

5. The method of claim 1, **characterized in that** the first wireless communication technology comprises a radio frequency identification technology or a near field communication technology; and/or,
the second wireless communication technology comprises at least one of a wireless medical telemetry service technology, a wireless local area network technology, a Bluetooth communication technology, and a ZigBee communication technology.

6. The method of claim 1, **characterized in that** the collecting device (10) comprises a first coil, and the receiving device (20) comprises a second coil, wherein the second coil sends a radio frequency signal at a preset frequency, the first coil receives the radio frequency signal after entering into an induction range and produces an induced current to activate the collecting device (10), and after the collecting device (10) is activated, the collecting device (10) acquires the configuration data transmitted by the receiving device (20).

7. The method of claim 1, **characterized in that** when the collecting device (10) determines that the first pairing information matches the second pairing information, transmitting, by using the second wireless communication, to the receiving device (20) the physiological signal or the physiological information generated according to the physiological signal;
when the collecting device (10) determines that the first pairing information does not match the second pairing information, discarding or ignoring, by the collecting device (10), subsequent data sent by the receiving device (20), and/or prohibiting the collecting device (10) from sending to the receiving device (20) the physiological signal or the physiological information generated according to the physiological signal.

8. The method of claim 1, **characterized in that** the collecting device (10) is a portable collecting device, and the collecting device (10) is used to display the physiological signal or the physiological information generated according to the physiological signal.

9. A method for communicating between a collecting device (10) and a receiving device (20), the collecting device (10) is configured to be connected to a patient to collect a physiological signal of the patient, and the method comprises:
transmitting, by the receiving device (20), configuration data to the collecting device (10) by using a first wireless communication technology, wherein the configuration data is used to determine first pairing information; and
communicating, by the receiving device (20), with the collecting device (10) by using a second wireless communication technology, to transmit second pairing information to the collecting device (10), so that, after determining that the first pairing information matches the second pairing information, the collecting device (10) is allowed to send to the receiving device (20) the physiological signal or physiological information generated according to the physiological signal;
**characterized in that** the configuration data comprises a frequency parameter corresponding to a communication channel, and the frequency parameter corresponding to the communication channel in the configuration data is part of frequency parameters in communication channels supported by the collecting device (10); the configuration data is used to determine the communication channel and further set the communication channel using the second wireless communication technology according to the frequency parameter in the configuration data;
the method, executed by the receiving device (20), further comprises: communicating with the collecting device (10) on the communication channel corresponding to the frequency parameter to transmit the second pairing information.

10. The method of claim 9, **characterized in that** the first pairing information is used to uniquely identify a control authority of the collecting device (10); and the method further comprises:
transmitting, by the receiving device (20), a control instruction to the collecting device (10) on the communication channel by using the second wireless communication technology, so that after the collecting device (10) determines the first pairing information matches the second pairing information, executing, by the collecting device (10), the control instruction.

11. The method of claim 9, **characterized in that** the configuration data comprises the first pairing information and the frequency parameter corresponding to the communication channel, the first pairing information is generated by the receiving device (20) according to a number of times that pairing has been performed.

12. The method of claim 9, **characterized in that** the configuration data comprises a receiving device identification code used to generate the first pairing information and a frequency parameter corresponding to the communication channel; and the first pairing information is generated by the collecting device (10) according to the receiving device identification code.

13. A collecting device (10), comprising:
a collecting unit (110) configured to be connected to a patient to collect a physiological signal of the patient;
a first wireless unit (120) configured to wirelessly communicate with a receiving device (20) by using a first wireless communication technology;
a second wireless unit (140) configured to wirelessly communicate with the receiving device (20) by using a second wireless communication technology;
a first processing unit (130) configured to acquire configuration data from the receiving device (20) by using the first wireless unit (120), determine first pairing information according to the configuration data, and communicate with the receiving device (20) by using the second wireless unit (140), to acquire second pairing information transmitted by the receiving device (20); and when the first processing unit determines the first pairing information matches the second pairing information, the collecting device (10) is allowed to send the collected physiological signal or physiological information generated according to the physiological signal to the receiving device (20);
**characterized in that** the configuration data comprises a frequency parameter corresponding to a communication channel, and the first processing unit (130) is further configured to:
determine the communication channel according to the configuration data, set the communication channel using the second wireless communication technology according to the frequency parameter in the configuration data, and acquire the second pairing information that is transmitted by the receiving device (20) on the communication channel corresponding to the frequency parameter.

14. A receiving device (20), comprising:
a third wireless unit (210) configured to wirelessly communicate between a collecting device (10) and using a first wireless communication technology, the collecting device (10) being configured to be connected to a patient to collect a physiological signal of the patient;
a fourth wireless unit (230) configured to wirelessly communicate with the collecting device (10) by using a second wireless communication technology; and
a second processing unit (220) configured to transmit configuration data to the collecting device (10) by using the third wireless unit (210), wherein the configuration data is used to determine first pairing information; and communicate with the collecting device (10) by using the fourth wireless unit (230), to transmit second pairing information to the collecting device (10), so that after the collecting device (10) determines the first pairing information matches the second pairing information, the collecting device (10) is allowed to send to the receiving device (20) the collected physiological signal or physiological information generated according to the physiological signal;
**characterized in that** the configuration data comprises a frequency parameter corresponding to a communication channel, and the frequency parameter corresponding to the communication channel in the configuration data is part of frequency parameters in communication channels supported by the fourth wireless unit (230); the configuration data is used to determine the communication channel and further set the communication channel using the second wireless communication technology according to the frequency parameter in the configuration data;
the second processing unit (220) is further configured to: communicate with the collecting device (10) on the communication channel corresponding to the frequency parameter to transmit the second pairing information.

15. A monitor, comprising at least one collecting device (10) and a receiving device (20), wherein
the collecting device (10) is configured to be connected to a patient to collect a physiological signal of the patient, acquire configuration data from the receiving device (20) by using a first wireless communication technology, determine first pairing information according to the configuration data, and communicate with the receiving device (20) by using a second wireless communication technology, to acquire second pairing information transmitted by the receiving device (20); and when the collecting device (10) determines the first pairing information matches the second pairing information, the collecting device (10) is allowed to send to the receiving device (20) the physiological signal or physiological information generated according to the physiological signal; and
the receiving device (20) is configured to transmit the configuration data to the collecting device (10) by using the first wireless communication technology, wherein the configuration data is used to determine the first pairing information; and communicate with the collecting device (10) by using the second wireless communication technology, to transmit the second pairing information to the collecting device (10);
**characterized in that** the configuration data comprises a frequency parameter corresponding to a communication channel;
the collecting device (10) is further configured to determine the communication channel according to the configuration data, and set the communication channel using the second wireless communication technology according to the frequency parameter in the configuration data;
the collecting device (10) and the receiving device (20) are configured to communicate on the communication channel corresponding to the frequency parameter such that the collecting device (10) transmits the second pairing information on the communication channel.

## Patentansprüche

1. Ein Verfahren zur Kommunikation zwischen einer Empfangsvorrichtung (20) und einer Erfassungsvorrichtung (10), wobei die Erfassungsvorrichtung (10) so ausgelegt ist, dass sie mit einem Patienten verbunden wird, um ein physiologisches Signal des Patienten zu erfassen, und wobei das Verfahren Folgendes umfasst:
das Erfassen von Konfigurationsdaten von der Empfangsvorrichtung (20) durch die Erfassungsvorrichtung (10) unter Verwendung einer ersten drahtlosen Kommunikationstechnologie; und das Bestimmen erster Kopplungsinformationen gemäß den Konfigurationsdaten, wobei die Konfigurationsdaten einen Frequenzparameter umfassen, der einem Kommunikationskanal entspricht;
das Kommunizieren durch die Erfassungsvorrichtung (10) mit der Empfangsvorrichtung (20) unter Verwendung einer zweiten drahtlosen Kommunikationstechnologie, um von der Empfangsvorrichtung (20) übertragene zweite Kopplungsinformationen zu erfassen; und
das Ermitteln durch die Erfassungsvorrichtung (10), ob die ersten Kopplungsinformationen mit den zweiten Kopplungsinformationen übereinstimmen, und wenn die ersten Kopplungsinformationen mit den zweiten Kopplungsinformationen übereinstimmen, es der Erfassungsvorrichtung (10) gestattet ist, das physiologische Signal oder die gemäß dem physiologischen Signal erzeugten physiologischen Informationen an die Empfangsvorrichtung (20) zu senden, so dass die Empfangsvorrichtung (20) das physiologische Signal oder die physiologischen Informationen auf dem dem Frequenzparameter entsprechenden Kommunikationskanal empfängt;
wobei das von der Erfassungsvorrichtung (10) ausgeführte Verfahren ferner Folgendes umfasst:
das Ermitteln des Kommunikationskanals gemäß den Konfigurationsdaten, das Einstellen des Kommunikationskanals unter Verwendung der zweiten drahtlosen Kommunikationstechnologie gemäß dem Frequenzparameter in den Konfigurationsdaten und das Erfassen der zweiten Kopplungsinformationen, die von der Empfangseinrichtung (20) auf dem dem Frequenzparameter entsprechenden Kommunikationskanal übertragen wird.

2. Das Verfahren nach Anspruch 1, **dadurch gekennzeichnet, dass** die ersten Kopplungsinformationen verwendet werden, um eine Steuerinstanz des Erfassungsgeräts (10) eindeutig zu identifizieren; wobei das Verfahren ferner Folgendes umfasst:
das Kommunizieren durch die Erfassungsvorrichtung (10) mit der Empfangsvorrichtung (20) auf dem Kommunikationskanal unter Verwendung der zweiten drahtlosen Kommunikationstechnologie, um eine von der Empfangsvorrichtung (20) gesendete Steueranweisung zu erfassen; und
wenn die Erfassungsvorrichtung (10) feststellt, dass die ersten Kopplungsinformationen mit den zweiten Kopplungsinformationen übereinstimmen, das Ausführen der Steueranweisung durch die Erfassungsvorrichtung (10).

3. Das Verfahren nach Anspruch 1, **dadurch gekennzeichnet, dass** die Konfigurationsdaten die ersten Kopplungsinformationen und den dem Kommunikationskanal entsprechenden Frequenzparameter umfassen, wobei die ersten Kopplungsinformationen von der Empfangsvorrichtung (20) erzeugt werden; der dem Kommunikationskanal in den Konfigurationsdaten entsprechende Frequenzparameter Teil der Frequenzparameter in den von der Erfassungsvorrichtung (10) unterstützten Kommunikationskanälen ist, so dass die Empfangsvorrichtung (20) in der Lage ist, drahtlos mit verschiedenen Erfassungsvorrichtungen (10) auf unterschiedlichen Kommunikationskanälen zu kommunizieren; und
das Bestimmen der ersten Kopplungsinformationen und eines Kommunikationskanals durch die Erfassungsvorrichtung (10) gemäß den Konfigurationsdaten Folgendes umfasst:
das Bestimmen der ersten Kopplungsinformationen durch das Erfassungsgerät (10) gemäß den ersten Kopplungsinformationen in den Konfigurationsdaten und das Bestimmen des Kommunikationskanals gemäß dem dem Kommunikationskanal entsprechenden Frequenzparameter.

4. Das Verfahren nach Anspruch 1, **dadurch gekennzeichnet, dass** die Konfigurationsdaten einen Empfangsgeräte-Identifikationscode, der zur Erzeugung der ersten Kopplungsinformationen verwendet wird, und den dem Kommunikationskanal entsprechenden Frequenzparameter umfassen; wobei der dem Kommunikationskanal entsprechende Frequenzparameter in den Konfigurationsdaten Teil der Frequenzparameter in den von der Erfassungsvorrichtung (10) unterstützten Kommunikationskanälen ist, so dass die Empfangsvorrichtung (20) in der Lage ist, drahtlos mit verschiedenen Erfassungsvorrichtungen (10) auf unterschiedlichen Kommunikationskanälen zu kommunizieren; und
das Bestimmen erster Kopplungsinformationen und eines Kommunikationskanals durch das Erfassungsgerät (10) gemäß den Konfigurationsdaten Folgendes umfasst:
das Erzeugen der ersten Kopplungsinformationen durch das Erfassungsgerät (10) gemäß dem Identifikationscode des Empfangsgeräts und das Bestimmen des Kommunikationskanals gemäß dem dem Kommunikationskanal entsprechenden Frequenzparameter; und dass das Verfahren ferner Folgendes umfasst:
die Übertragung der ersten Kopplungsinformationen durch die Erfassungsvorrichtung (10) an die Empfangsvorrichtung (20) unter Verwendung der ersten drahtlosen Kommunikationstechnologie.

5. Das Verfahren nach Anspruch 1, **dadurch gekennzeichnet, dass** die erste drahtlose Kommunikationstechnologie eine Radiofrequenz-Identifikationstechnologie oder eine Nahfeldkommunikationstechnologie umfasst; und/oder,
die zweite drahtlose Kommunikationstechnologie mindestens eine der folgenden Technologien umfasst: eine Technologie für drahtlose medizinische Telemetriedienste, eine Technologie für drahtlose lokale Netzwerke, eine Bluetooth-Kommunikationstechnologie und eine ZigBee-Kommunikationstechnologie.

6. Das Verfahren nach Anspruch 1, **dadurch gekennzeichnet, dass** die Erfassungsvorrichtung (10) eine erste Spule umfasst und die Empfangsvorrichtung (20) eine zweite Spule umfasst, wobei die zweite Spule ein Hochfrequenzsignal mit einer voreingestellten Frequenz sendet, die erste Spule das Hochfrequenzsignal nach Eintritt in einen Induktionsbereich empfängt und einen induzierten Strom erzeugt, um die Erfassungsvorrichtung (10) zu aktivieren, und nachdem die Erfassungsvorrichtung (10) aktiviert wurde, die Erfassungsvorrichtung (10) die von der Empfangsvorrichtung (20) übertragenen Konfigurationsdaten erfasst.

7. Das Verfahren nach Anspruch 1, **dadurch gekennzeichnet, dass**, wenn die Erfassungsvorrichtung (10) feststellt, dass die ersten Kopplungsinformationen mit den zweiten Kopplungsinformationen übereinstimmen, unter Verwendung der zweiten drahtlosen Kommunikation das physiologische Signal oder die gemäß dem physiologischen Signal erzeugten physiologischen Informationen an die Empfangsvorrichtung (20) übertragen werden;
wenn die Erfassungsvorrichtung (10) feststellt, dass die ersten Kopplungsinformationen nicht mit den zweiten Kopplungsinformationen übereinstimmen, das Verwerfen oder Ignorieren nachfolgender, von der Empfangsvorrichtung (20) gesendeter Daten durch die Erfassungsvorrichtung (10) und/oder das Unterbinden des Sendens des physiologischen Signals oder der gemäß dem physiologischen Signal erzeugten physiologischen Informationen von der Erfassungsvorrichtung (10) an die Empfangsvorrichtung (20).

8. Das Verfahren nach Anspruch 1, **dadurch gekennzeichnet, dass** die Erfassungsvorrichtung (10) eine tragbare Erfassungsvorrichtung ist und die Erfassungsvorrichtung (10) dazu verwendet wird, das physiologische Signal oder die gemäß dem physiologischen Signal erzeugten physiologischen Informationen anzuzeigen.

9. Das Verfahren zur Kommunikation zwischen einer Erfassungsvorrichtung (10) und einer Empfangsvorrichtung (20), wobei die Erfassungsvorrichtung (10) so ausgelegt ist, dass sie mit einem Patienten verbunden wird, um ein physiologisches Signal des Patienten zu erfassen, und wobei das Verfahren Folgendes umfasst:
das Senden von Konfigurationsdaten durch die Empfangsvorrichtung (20) an die Erfassungsvorrichtung (10) unter Verwendung einer ersten drahtlosen Kommunikationstechnologie, wobei die Konfigurationsdaten zur Bestimmung erster Kopplungsinformationen verwendet werden; und
das Kommunizieren durch die Empfangsvorrichtung (20) mit der Erfassungsvorrichtung (10) unter Verwendung einer zweiten drahtlosen Kommunikationstechnologie, um zweite Kopplungsinformationen an die Erfassungsvorrichtung (10) zu übertragen, so dass die Erfassungsvorrichtung (10), nachdem festgestellt wurde, dass die ersten Kopplungsinformationen mit den zweiten Kopplungsinformationen übereinstimmen, das physiologische Signal oder die gemäß dem physiologischen Signal erzeugten physiologischen Informationen an die Empfangsvorrichtung (20) senden darf;
**dadurch gekennzeichnet, dass** die Konfigurationsdaten einen einem Kommunikationskanal entsprechenden Frequenzparameter umfassen und der dem Kommunikationskanal in den Konfigurationsdaten entsprechende Frequenzparameter Teil der Frequenzparameter in den vom Erfassungsgerät (10) unterstützten Kommunikationskanälen ist; die Konfigurationsdaten verwendet werden, um den Kommunikationskanal zu bestimmen und den Kommunikationskanal unter Verwendung der zweiten drahtlosen Kommunikationstechnologie gemäß dem Frequenzparameter in den Konfigurationsdaten weiter einzustellen;
das von der Empfangsvorrichtung (20) ausgeführte Verfahren ferner Folgendes umfasst: die Kommunikation mit der Erfassungsvorrichtung (10) über den dem Frequenzparameter entsprechenden Kommunikationskanal, um die zweiten Kopplungsinformationen zu übertragen.

10. Das Verfahren nach Anspruch 9, **dadurch gekennzeichnet, dass** die ersten Kopplungsinformationen verwendet werden, um eine Steuerinstanz der Erfassungsvorrichtung (10) eindeutig zu identifizieren; und das Verfahren ferner Folgendes umfasst:
das Senden einer Steueranweisung durch die Empfangsvorrichtung (20) an die Erfassungsvorrichtung (10) über den Kommunikationskanal unter Verwendung der zweiten drahtlosen Kommunikationstechnologie, so dass, nachdem die Erfassungsvorrichtung (10) festgestellt hat, dass die ersten Kopplungsinformationen mit den zweiten Kopplungsinformationen übereinstimmen, die Erfassungsvorrichtung (10) die Steueranweisung ausführt.

11. Das Verfahren nach Anspruch 9, **dadurch gekennzeichnet, dass** die Konfigurationsdaten die ersten Kopplungsinformationen und den dem Kommunikationskanal entsprechenden Frequenzparameter umfassen, wobei die ersten Kopplungsinformationen von der Empfangsvorrichtung (20) entsprechend der Anzahl der durchgeführten Kopplungsvorgänge erzeugt werden.

12. Das Verfahren nach Anspruch 9, **dadurch gekennzeichnet, dass** die Konfigurationsdaten einen Identifikationscode der Empfangsvorrichtung, der zur Erzeugung der ersten Kopplungsinformationen verwendet wird, und einen dem Kommunikationskanal entsprechenden Frequenzparameter umfassen; und dass die ersten Kopplungsinformationen von der Erfassungsvorrichtung (10) entsprechend dem Identifikationscode der Empfangsvorrichtung erzeugt werden.

13. Eine Erfassungsvorrichtung (10), die Folgendes umfasst:
eine Erfassungseinheit (110), die so ausgelegt ist, dass sie mit einem Patienten verbunden wird, um ein physiologisches Signal des Patienten zu erfassen;
eine erste Funkeinheit (120), die so ausgelegt ist, dass sie unter Verwendung einer ersten Funkkommunikationstechnologie drahtlos mit einem Empfangsgerät (20) kommuniziert;
eine zweite Funkeinheit (140), die so ausgelegt ist, dass sie unter Verwendung einer zweiten Funkkommunikationstechnologie drahtlos mit der Empfangsvorrichtung (20) kommuniziert;
eine erste Verarbeitungseinheit (130), die so ausgelegt ist, dass sie Konfigurationsdaten von der Empfangsvorrichtung (20) unter Verwendung der ersten Funkeinheit (120) erfasst, erste Kopplungsinformationen gemäß den Konfigurationsdaten ermittelt und mit der Empfangsvorrichtung (20) unter Verwendung der zweiten Funkeinheit (140) kommuniziert, um von der Empfangsvorrichtung (20) übertragene zweite Kopplungsinformationen zu erfassen; und wenn die erste Verarbeitungseinheit feststellt, dass die ersten Kopplungsinformationen mit den zweiten Kopplungsinformationen übereinstimmen, es dem Erfassungsgerät (10) gestattet wird, das erfasste physiologische Signal oder die gemäß dem physiologischen Signal erzeugten physiologischen Informationen an das Empfangsgerät (20) zu senden;
**dadurch gekennzeichnet, dass** die Konfigurationsdaten einen einem Kommunikationskanal entsprechenden Frequenzparameter umfassen und die erste Verarbeitungseinheit (130) ferner so ausgelegt ist, dass sie:
den Kommunikationskanal gemäß den Konfigurationsdaten bestimmt, den Kommunikationskanal unter Verwendung der zweiten drahtlosen Kommunikationstechnologie gemäß dem Frequenzparameter in den Konfigurationsdaten einstellt und die zweiten Kopplungsinformationen erfasst, die von der Empfangsvorrichtung (20) auf dem dem Frequenzparameter entsprechenden Kommunikationskanal übertragen wird.

14. Ein Empfangsgerät (20), das Folgendes umfassend:
eine dritte Funkeinheit (210), die so ausgelegt ist, dass sie drahtlos mit einem Erfassungsgerät (10) unter Verwendung einer ersten drahtlosen Kommunikationstechnologie kommuniziert, wobei das Erfassungsgerät (10) so ausgelegt ist, dass es an einen Patienten angeschlossen wird, um ein physiologisches Signal des Patienten zu erfassen;
eine vierte Funkeinheit (230), die so ausgelegt ist, dass sie unter Verwendung einer zweiten Funkkommunikationstechnologie drahtlos mit der Erfassungsvorrichtung (10) kommuniziert; und
eine zweite Verarbeitungseinheit (220), die so ausgelegt ist, dass sie Konfigurationsdaten unter Verwendung der dritten Funkeinheit (210) an das Erfassungsgerät (10) überträgt, wobei die Konfigurationsdaten zur Ermittlung erster Kopplungsinformationen verwendet werden; und zur Kommunikation mit der Erfassungsvorrichtung (10) unter Verwendung der vierten Funkeinheit (230), um zweite Kopplungsinformationen an die Erfassungsvorrichtung (10) zu übertragen, sodass die Erfassungsvorrichtung (10), nachdem sie festgestellt hat, dass die ersten Kopplungsinformationen mit den zweiten Kopplungsinformationen übereinstimmen, das erfasste physiologische Signal oder die gemäß dem physiologischen Signal erzeugten physiologischen Informationen an die Empfangsvorrichtung (20) senden darf;
**dadurch gekennzeichnet, dass** die Konfigurationsdaten einen einem Kommunikationskanal entsprechenden Frequenzparameter umfassen und der dem Kommunikationskanal entsprechende Frequenzparameter in den Konfigurationsdaten Teil der Frequenzparameter in den von der vierten Funkeinheit (230) unterstützten Kommunikationskanälen ist; die Konfigurationsdaten dazu verwendet werden, den Kommunikationskanal zu bestimmen und den Kommunikationskanal unter Verwendung der zweiten Funkkommunikationstechnologie gemäß dem Frequenzparameter in den Konfigurationsdaten weiter einzustellen;
die zweite Verarbeitungseinheit (220) ferner so ausgelegt ist, dass sie: mit der Erfassungsvorrichtung (10) über den dem Frequenzparameter entsprechenden Kommunikationskanal kommuniziert, um die zweiten Kopplungsinformationen zu übertragen.

15. Ein Monitor, der mindestens eine Erfassungsvorrichtung (10) und eine Empfangsvorrichtung (20) umfasst, wobei
die Erfassungsvorrichtung (10) so ausgelegt ist, dass sie an einen Patienten angeschlossen wird, um ein physiologisches Signal des Patienten zu erfassen, Konfigurationsdaten von der Empfangsvorrichtung (20) unter Verwendung einer ersten drahtlosen Kommunikationstechnologie zu erfassen, erste Kopplungsinformationen gemäß den Konfigurationsdaten zu ermitteln und mit der Empfangsvorrichtung (20) unter Verwendung einer zweiten drahtlosen Kommunikationstechnologie zu kommunizieren, um von der Empfangsvorrichtung (20) übertragene zweite Kopplungsinformationen zu erfassen; und wenn die Erfassungsvorrichtung (10) feststellt, dass die ersten Kopplungsinformationen mit den zweiten Kopplungsinformationen übereinstimmen, es der Erfassungsvorrichtung (10) gestattet ist, das physiologische Signal oder die gemäß dem physiologischen Signal erzeugten physiologischen Informationen an die Empfangsvorrichtung (20) zu senden; und
die Empfangsvorrichtung (20) so ausgelegt ist, dass sie die Konfigurationsdaten unter Verwendung der ersten drahtlosen Kommunikationstechnologie an die Erfassungsvorrichtung (10) überträgt, wobei die Konfigurationsdaten zur Bestimmung der ersten Kopplungsinformationen verwendet werden; und mit der Erfassungsvorrichtung (10) unter Verwendung der zweiten drahtlosen Kommunikationstechnologie kommuniziert, um die zweiten Kopplungsinformationen an die Erfassungsvorrichtung (10) zu übertragen;
**dadurch gekennzeichnet, dass** die Konfigurationsdaten einen Frequenzparameter umfassen, der einem Kommunikationskanal entspricht;
die Erfassungsvorrichtung (10) ferner so konfiguriert ist, dass sie den Kommunikationskanal gemäß den Konfigurationsdaten ermittelt und den Kommunikationskanal unter Verwendung der zweiten drahtlosen Kommunikationstechnologie entsprechend dem Frequenzparameter in den Konfigurationsdaten einstellt;
die Erfassungsvorrichtung (10) und die Empfangsvorrichtung (20) so ausgelegt sind, dass sie über den dem Frequenzparameter entsprechenden Kommunikationskanal kommunizieren, sodass die Erfassungsvorrichtung (10) die zweite Kopplungsinformation über den Kommunikationskanal überträgt.

## Revendications

1. Méthode de communication entre un dispositif de réception (20) et un dispositif de collecte (10), le dispositif de collecte (10) est configuré pour être relié à un patient pour collecter un signal physiologique du patient, et la méthode comprend les étapes consistant à :
acquérir, par le biais du dispositif de collecte (10), des données de configuration auprès du dispositif de réception (20) en utilisant une première technologie de communication sans fil, et déterminer une première information d'appariement selon les données de configuration, où les données de configuration comprennent un paramètre de fréquence correspondant à un canal de communication ;
communiquer, par le biais du dispositif de collecte (10), avec le dispositif de réception (20) en utilisant une seconde technologie de communication sans fil, pour acquérir une seconde information d'appariement transmise par le dispositif de réception (20) ; et
déterminer, par le biais du dispositif de collecte (10), si la première information d'appariement correspond à la seconde information d'appariement, et lorsque la première information d'appariement correspond à la seconde information d'appariement, le dispositif de collecte (10) est autorisé à envoyer au dispositif de réception (20) le signal physiologique ou l'information physiologique générée selon le signal physiologique de telle sorte que le dispositif de réception (20) reçoit le signal physiologique ou l'information physiologique sur le canal de communication correspondant au paramètre de fréquence ;
où la méthode, exécutée par le dispositif de collecte (10), comprend en outre :
le fait de déterminer le canal de communication selon les données de configuration, d'établir le canal de communication en utilisant la seconde technologie de communication sans fil selon le paramètre de fréquence dans les données de configuration, d'acquérir la seconde information d'appariement qui est transmise par le dispositif de réception (20) sur le canal de communication correspondant au paramètre de fréquence.

2. Méthode selon la revendication 1, **caractérisée en ce que** la première information d'appariement est utilisée pour identifier de manière univoque une autorité de contrôle du dispositif de collecte (10) ; la méthode comprend en outre :
le fait de communiquer, par le biais du dispositif de collecte (10), avec le dispositif de réception (20) sur le canal de communication en utilisant la seconde technologie de communication sans fil, pour acquérir une instruction de contrôle transmise par le dispositif de réception (20) ; et
lorsque le dispositif de collecte (10) détermine que la première information d'appariement correspond à la seconde information d'appariement, le fait d'exécuter, par le dispositif de collecte (10), l'instruction de contrôle.

3. Méthode selon la revendication 1, **caractérisée en ce que** les données de configuration comprennent la première information d'appariement et le paramètre de fréquence correspondant au canal de communication, la première information d'appariement étant générée par le dispositif de réception (20) ; le paramètre de fréquence correspondant au canal de communication dans les données de configuration fait partie de paramètres de fréquence dans des canaux de communication pris en charge par le dispositif de collecte (10), de sorte que le dispositif de réception (20) est en mesure de communiquer de manière sans fil avec différents dispositifs de collecte (10) sur différents canaux de communication ; et
le fait de déterminer, par le biais du dispositif de collecte (10), une première information d'appariement et un canal de communication selon les données de configuration comprend :
le fait de déterminer, par le biais du dispositif de collecte (10), la première information d'appariement selon la première information d'appariement dans les données de configuration, et de déterminer le canal de communication selon le paramètre de fréquence correspondant au canal de communication.

4. Méthode selon la revendication 1, **caractérisée en ce que** les données de configuration comprennent un code d'identification de dispositif de réception utilisé pour générer la première information d'appariement, et le paramètre de fréquence correspondant au canal de communication ; le paramètre de fréquence correspondant au canal de communication dans les données de configuration fait partie de paramètres de fréquence dans des canaux de communication pris en charge par le dispositif de collecte (10), de sorte que le dispositif de réception (20) est en mesure de communiquer de manière sans fil avec différents dispositifs de collecte (10) sur différents canaux de communication ; et
le fait de déterminer, par le biais du dispositif de collecte (10), une première information d'appariement et un canal de communication selon les données de configuration comprend :
le fait de générer, par le biais du dispositif de collecte (10), la première information d'appariement selon le code d'identification de dispositif de réception, et de déterminer le canal de communication selon le paramètre de fréquence correspondant au canal de communication ; et la méthode comprend en outre :
le fait de transmettre, par le biais du dispositif de collecte (10), la première information d'appariement au dispositif de réception (20) en utilisant la première technologie de communication sans fil.

5. Méthode selon la revendication 1, **caractérisée en ce que** la première technologie de communication sans fil comprend une technologie d'identification par radiofréquence ou une technologie de communication en champ proche ; et/ou
la seconde technologie de communication sans fil comprend au moins l'une d'une technologie de service de télémétrie médicale sans fil, d'une technologie de réseau local sans fil, d'une technologie de communication Bluetooth, et d'une technologie de communication ZigBee.

6. Méthode selon la revendication 1, **caractérisée en ce que** le dispositif de collecte (10) comprend une première bobine, et le dispositif de réception (20) comprend une seconde bobine, où la seconde bobine envoie un signal radiofréquence à une fréquence préétablie, la première bobine reçoit le signal radiofréquence après être entrée dans une plage d'induction et produit un courant induit pour activer le dispositif de collecte (10), et après que le dispositif de collecte (10) a été activé, le dispositif de collecte (10) acquiert les données de configuration transmises par le dispositif de réception (20).

7. Méthode selon la revendication 1, **caractérisée par**, lorsque le dispositif de collecte (10) détermine que la première information d'appariement correspond à la seconde information d'appariement, le fait de transmettre, en utilisant la seconde technologie de communication sans fil, au dispositif de réception (20) le signal physiologique ou l'information physiologique générée selon le signal physiologique ;
lorsque le dispositif de collecte (10) détermine que la première information d'appariement ne correspond pas à la seconde information d'appariement, le fait de rejeter ou d'ignorer, par le dispositif de collecte (10), des données suivantes envoyées par le dispositif de réception (20), et/ou le fait d'interdire au dispositif de collecte (10) d'envoyer au dispositif de réception (20) le signal physiologique ou l'information physiologique générée selon le signal physiologique.

8. Méthode selon la revendication 1, **caractérisée en ce que** le dispositif de collecte (10) est un dispositif de collecte portable, et le dispositif de collecte (10) est utilisé pour afficher le signal physiologique ou l'information physiologique générée selon le signal physiologique.

9. Méthode de communication entre un dispositif de collecte (10) et un dispositif de réception (20), le dispositif de collecte (10) est configuré pour être relié à un patient pour collecter un signal physiologique du patient, et la méthode comprend les étapes consistant à :
transmettre, par le biais du dispositif de réception (20), des données de configuration au dispositif de collecte (10) en utilisant une première technologie de communication sans fil, où les données de configuration sont utilisées pour déterminer une première information d'appariement ; et
communiquer, par le biais du dispositif de réception (20), avec le dispositif de collecte (10) en utilisant une seconde technologie de communication sans fil, pour transmettre une seconde information d'appariement au dispositif de collecte (10), de sorte que, après avoir déterminé que la première information d'appariement correspond à la seconde information d'appariement, le dispositif de collecte (10) est autorisé à envoyer au dispositif de réception (20) le signal physiologique ou l'information physiologique générée selon le signal physiologique ;
**caractérisée en ce que** les données de configuration comprennent un paramètre de fréquence correspondant à un canal de communication, et le paramètre de fréquence correspondant au canal de communication dans les données de configuration fait partie de paramètres de fréquence dans des canaux de communication pris en charge par le dispositif de collecte (10) ; les données de configuration sont utilisées pour déterminer le canal de communication et en outre établir le canal de communication en utilisant la seconde technologie de communication sans fil selon le paramètre de fréquence dans les données de configuration ;
la méthode, exécutée par le dispositif de réception (20), comprend en outre : le fait communiquer avec le dispositif de collecte (10) sur le canal de communication correspondant au paramètre de fréquence pour transmettre la seconde information d'appariement.

10. Méthode selon la revendication 9, **caractérisée en ce que** la première information d'appariement est utilisée pour identifier de manière univoque une autorité de contrôle du dispositif de collecte (10) ; et la méthode comprend en outre :
le fait de transmettre, par le biais du dispositif de réception (20), une instruction de contrôle au dispositif de collecte (10) sur le canal de communication en utilisant la seconde technologie de communication sans fil, de sorte à, après que le dispositif de collecte (10) a déterminé que la première information d'appariement correspond à la seconde information d'appariement, exécuter, par le dispositif de collecte (10), l'instruction de contrôle.

11. Méthode selon la revendication 9, **caractérisée en ce que** les données de configuration comprennent la première information d'appariement et le paramètre de fréquence correspondant au canal de communication, la première information d'appariement est générée par le dispositif de réception (20) selon un nombre de fois où l'appariement a été effectué.

12. Méthode selon la revendication 9, **caractérisée en ce que** les données de configuration comprennent un code d'identification de dispositif de réception utilisé pour générer la première information d'appariement et un paramètre de fréquence correspondant au canal de communication ; et la première information d'appariement est générée par le dispositif de collecte (10) selon le code d'identification de dispositif de réception.

13. Dispositif de collecte (10), comprenant :
une unité de collecte (110) configurée pour être reliée à un patient pour collecter un signal physiologique du patient ;
une première unité sans fil (120) configurée pour communiquer de manière sans fil avec un dispositif de réception (20) en utilisant une première technologie de communication sans fil ;
une deuxième unité sans fil (140) configurée pour communiquer de manière sans fil avec le dispositif de réception (20) en utilisant une seconde technologie de communication sans fil ;
une première unité de traitement (130) configurée pour acquérir des données de configuration auprès du dispositif de réception (20) en utilisant la première unité sans fil (120), déterminer une première information d'appariement selon les données de configuration, et communiquer avec le dispositif de réception (20) en utilisant la deuxième unité sans fil (140), pour acquérir une seconde information d'appariement transmise par le dispositif de réception (20) ; et lorsque la première unité de traitement détermine que la première information d'appariement correspond à la seconde information d'appariement, le dispositif de collecte (10) est autorisé à envoyer le signal physiologique collecté ou l'information physiologique générée selon le signal physiologique au dispositif de réception (20) ;
**caractérisé en ce que** les données de configuration comprennent un paramètre de fréquence correspondant à un canal de communication, et la première unité de traitement (130) est en outre configurée pour :
déterminer le canal de communication selon les données de configuration, établir le canal de communication en utilisant la seconde technologie de communication sans fil selon le paramètre de fréquence dans les données de configuration, et acquérir la seconde information d'appariement qui est transmise par le dispositif de réception (20) sur le canal de communication correspondant au paramètre de fréquence.

14. Dispositif de réception (20), comprenant :
une troisième unité sans fil (210) configurée pour communiquer de manière sans fil avec un dispositif de collecte (10) et en utilisant une première technologie de communication sans fil, le dispositif de collecte (10) étant configuré pour être relié à un patient pour collecter un signal physiologique du patient ;
une quatrième unité sans fil (230) configurée pour communiquer de manière sans fil avec le dispositif de collecte (10) en utilisant une seconde technologie de communication sans fil ; et
une seconde unité de traitement (220) configurée pour transmettre des données de configuration au dispositif de collecte (10) en utilisant la troisième unité sans fil (210), où les données de configuration sont utilisées pour déterminer une première information d'appariement ; et communiquer avec le dispositif de collecte (10) en utilisant la quatrième unité sans fil (230), pour transmettre une seconde information d'appariement au dispositif de collecte (10), de sorte qu'après que le dispositif de collecte (10) a déterminé que la première information d'appariement correspond à la seconde information d'appariement, le dispositif de collecte (10) est autorisé à envoyer au dispositif de réception (20) le signal physiologique collecté ou l'information physiologique générée selon le signal physiologique ;
**caractérisé en ce que** les données de configuration comprennent un paramètre de fréquence correspondant à un canal de communication, et le paramètre de fréquence correspondant au canal de communication dans les données de configuration fait partie de paramètres de fréquence dans des canaux de communication pris en charge par la quatrième unité sans fil (230) ; les données de configuration sont utilisées pour déterminer le canal de communication et en outre établir le canal de communication en utilisant la seconde technologie de communication sans fil selon le paramètre de fréquence dans les données de configuration ;
la seconde unité de traitement (220) est en outre configurée pour : communiquer avec le dispositif de collecte (10) sur le canal de communication correspondant au paramètre de fréquence pour transmettre la seconde information d'appariement.

15. Moniteur, comprenant au moins un dispositif de collecte (10) et un dispositif de réception (20), où
le dispositif de collecte (10) est configuré pour être relié à un patient pour collecter un signal physiologique du patient, acquérir des données de configuration auprès du dispositif de réception (20) en utilisant une première technologie de communication sans fil, déterminer une première information d'appariement selon les données de configuration, et communiquer avec le dispositif de réception (20) en utilisant une seconde technologie de communication sans fil, pour acquérir une seconde information d'appariement transmise par le dispositif de réception (20) ; et lorsque le dispositif de collecte (10) détermine que la première information d'appariement correspond à la seconde information d'appariement, le dispositif de collecte (10) est autorisé à envoyer au dispositif de réception (20) le signal physiologique ou l'information physiologique générée selon le signal physiologique ; et
le dispositif de réception (20) est configuré pour transmettre les données de configuration au dispositif de collecte (10) en utilisant la première technologie de communication sans fil, où les données de configuration sont utilisées pour déterminer la première information d'appariement ; et communiquer avec le dispositif de collecte (10) en utilisant la seconde technologie de communication sans fil, pour transmettre la seconde information d'appariement au dispositif de collecte (10) ;
**caractérisé en ce que** les données de configuration comprennent un paramètre de fréquence correspondant à un canal de communication ;
le dispositif de collecte (10) est en outre configuré pour déterminer le canal de communication selon les données de configuration, et établir le canal de communication en utilisant la seconde technologie de communication sans fil selon le paramètre de fréquence dans les données de configuration ;
le dispositif de collecte (10) et le dispositif de réception (20) sont configurés pour communiquer sur le canal de communication correspondant au paramètre de fréquence de telle sorte que le dispositif de collecte (10) transmet la seconde information d'appariement sur le canal de communication.
